(19) Europäisches Patentamt
European Patent Office
Office européen des brevets

(11) EP 1 600 146 A1

(12) **DEMANDE DE BREVET EUROPEEN**

(43) Date de publication:
**30.11.2005 Bulletin 2005/48**

(51) Int Cl.$^7$: **A61K 7/021**

(21) Numéro de dépôt: **05290962.9**

(22) Date de dépôt: **03.05.2005**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **25.05.2004 FR 0451026**

(71) Demandeur: **L'OREAL**
**75008 Paris (FR)**

(72) Inventeur: **Blin, Xavier**
**75015 Paris (FR)**

(74) Mandataire: **Chantraine, Sylvie Hélène**
**L'OREAL,**
**River Plaza,**
**25-29 Quai Aulagnier**
**92600 Asnieres-sur-Seine (FR)**

(54) **Produit cosmétique bicouche, ses utilisations et kit de maquillage contenant ce produit**

(57) L'invention se rapporte à un produit cosmétique contenant une première et une seconde compositions, la première composition comprenant une dispersion, dans une phase grasse liquide, de particules d'un polymère éthylénique greffé et la seconde composition comprenant un milieu cosmétiquement acceptable.

L'invention se rapporte également à un procédé de maquillage ainsi qu'à un kit de maquillage contenant ledit produit. Ce dernier est en particulier un rouge à lèvres, un mascara ou un vernis à ongles.

EP 1 600 146 A1

## Description

La présente invention se rapporte à un produit cosmétique comprenant au moins deux compositions qui peuvent être appliquées successivement sur la peau aussi bien du visage que du corps, sur les paupières inférieures et supérieures, sur les lèvres et sur les phanères comme les ongles, les sourcils, les cils ou les cheveux. La présente invention concerne également un procédé de maquillage du visage et du corps mettant en oeuvre ces deux compositions.

Chaque composition peut être un fond de teint, un fard à joues ou à paupières, un produit anti-cerne, un blush, une poudre libre ou compactée, un rouge à lèvres, un baume à lèvres, un brillant à lèvres, un crayon à lèvres ou à yeux, un mascara, un eye-liner, un vernis à ongles ou encore un produit de maquillage du corps ou de coloration de la peau.

Un objectif de la présente invention est de proposer une composition qui soit à la fois de bonne tenue et brillante. Un autre objectif de la présente invention est de proposer une composition qui soit dotée de propriétés de non transfert et de brillance. En particulier, les compositions selon l'invention ont une bonne tenue, sont non transfert et brillantes.

Une mauvaise tenue dans le temps peut se traduire en particulier par une mauvaise tenue de la couleur et/ou par une mauvaise tenue de la brillance dans le temps. Cette mauvaise tenue peut se caractériser par une modification de la couleur (virage, palissement) généralement par suite d'une interaction avec le sébum et/ou la sueur sécrétés par la peau dans le cas de fond de teint et de fard, ou d'une interaction avec la salive dans le cas des rouges à lèvres, ou d'une diminution de la brillance, dans le cas d'un vernis à ongles. Ceci oblige l'utilisateur à se remaquiller très souvent, ce qui peut constituer une perte de temps.

Les compositions de maquillage pour les lèvres et la peau dites « sans transfert » sont des compositions qui présentent l'avantage de former un dépôt qui ne se dépose pas, au moins en partie, sur les supports avec lesquels elles sont mises en contact (verre, vêtements, cigarette, tissus).

Les compositions sans transfert connues sont généralement à base de résines de silicone et d'huiles de silicone volatiles et, bien que présentant des propriétés de tenue améliorées, ont l'inconvénient de laisser sur la peau et les lèvres, après évaporation des huiles de silicone volatiles, un film qui devient inconfortable au cours du temps (sensation de dessèchement et de tiraillement), écartant un certain nombre de femmes de ce type de rouge à lèvres. En outre, ces compositions à base d'huiles volatiles de silicone et de résines siliconées conduisent à des films colorés mats. Or, les femmes sont aujourd'hui à la recherche de produits, notamment de coloration des lèvres ou des paupières, brillants, tout en étant de bonne tenue, et sans transfert.

Pour obtenir des produits de bonne tenue et non transfert, il est souvent utilisé des huiles volatiles ou des polymères en dispersion dans un solvant volatil. Cependant, ces produits ne sont pas suffisamment brillants. Il a donc été envisagé un produit cosmétique comprenant deux compositions, une première contenant un polymère en dispersion dans un solvant volatile, sur laquelle on applique une deuxième composition brillante et grasse (FR-A-2823101). La tenue de la couleur du maquillage obtenu avec ces compositions est bonne, mais la disparition de la deuxième composition au cours de la journée lorsqu'elle est portée entraîne une perte de la brillance, souhaitée par la consommatrice.

La société Kose a par ailleurs proposé dans sa demande de brevet JP-A-0 5 221 829 l'utilisation d'un gel à base de matières perfluorées, que l'on applique sur un film de rouge à lèvres de manière à éviter son transfert sur d'autres surfaces, le gel étant incompatible avec le film de rouge à lèvres.

On peut aussi citer la demande de brevet WO-A-97/17057 qui décrit une méthode pour augmenter les propriétés de tenue et de non transfert consistant à appliquer deux compositions l'une sur l'autre. La composition à appliquer possède un paramètre de solubilité global d'Hildebrand inférieur à 8,5 $(cal/cm^3)^{1/2}$, et la composition à appliquer à titre de surcouche doit contenir des huiles dont le coefficient de partage calculé ClogP est au moins égal à 13.

Le brevet US-A-6 001 374 propose un système de maquillage multicouche consistant à utiliser une composition contenant une résine soluble dans l'alcool et insoluble dans l'eau, que l'on peut appliquer comme couche de base ou en surcouche, et présentant l'avantage de ne pas tâcher un objet mis au contact du maquillage et de résister à l'eau et aux frottements, tout en ayant un certain brillant. Cependant, cette composition contient un alcool hydrosoluble, en particulier l'éthanol, composé qui présente un caractère irritant, desséchant pour la peau et plus spécialement pour les lèvres, et qui est particulièrement inconfortable lorsque la peau ou les lèvres sont abîmées.

Dans la demande WO 02/067877, il est décrit une méthode pour améliorer les propriétés esthétiques d'une composition non transfert consistant à appliquer une deuxième composition sur un film de composition non transfert. La deuxième composition ne doit pas interagir chimiquement avec la composition non transfert afin de ne pas altérer ses propriétés cosmétiques. Certains produits décrits dans ce document ont une odeur désagréable et sont collants. D'autres produits ne sont pas assez brillants.

La présente invention a pour but de proposer une nouvelle voie de formulation d'un produit cosmétique, en particulier un produit de maquillage, du type comportant deux compositions à appliquer successivement l'une après

l'autre. Ce produit cosmétique peut avantageusement présenter de bonnes propriétés de brillance, de non transfert et/ou de tenue.

C'est en particulier un objet de l'invention que de réaliser un tel produit cosmétique doté de bonnes propriétés de non transfert et de tenue de la brillance.

La présente invention a encore pour objet de proposer un produit cosmétique, en particulier un produit de maquillage, qui réunisse avantageusement à la fois les propriétés de « sans transfert », de non migration, de tenue de la couleur, de confort, de non dessèchement, de brillance et de tenue de la brillance dans le temps.

Le demandeur a constaté que ces objets pouvaient être atteints en associant une première composition comprenant une dispersion, dans une phase grasse liquide, de particules d'un polymère éthylénique greffé, et une seconde composition comprenant un milieu cosmétiquement acceptable. La seconde composition contient en particulier au moins une huile.

Ainsi, les compositions selon au moins certains des aspects de l'invention permettent d'obtenir un rendu cosmétique très brillant à l'application et dans le temps, qui ne migre pas, ne transfère pas, de bonne tenue, tout en étant confortable à l'application et dans le temps (non collant, non desséchant, pas de tiraillement).

En particulier, le produit de l'invention permet l'obtention de dépôts continus sur la peau ou les lèvres, non collants, de bonne couvrance, ayant un aspect très brillant, adapté au désir de la consommatrice, ne migrant pas, ne transférant pas et de bonne tenue, non huileux, ne desséchant pas la peau, les cheveux ou les lèvres sur lesquelles il est appliqué, aussi bien lors de l'application qu'au cours du temps. Il présente, en outre de bonnes propriétés de stabilité et permet ainsi une application homogène et esthétique.

On a de plus constaté que les compositions du produit selon l'invention présentent des qualités d'étalement et d'adhésion sur la peau, les lèvres ou les cils, particulièrement intéressantes, ainsi qu'un toucher onctueux et agréable.

Ces propriétés de tenue, de non transfert et de non migration, alliées à l'aspect brillant et non gras, en font un produit particulièrement approprié pour réaliser des produits de maquillage des lèvres tels que rouges à lèvres, brillants à lèvres, des yeux tels que mascara, eye-liners, fards à paupières, des ongles ou des cheveux.

Un objet de la présente invention est donc un produit cosmétique destiné à être appliqué sur la peau, notamment la peau du visage, du cou, les lèvres, les paupières, comprenant une première et une seconde compositions, la première composition contenant une dispersion, dans une phase grasse liquide, de particules d'un polymère éthylénique greffé, et la seconde composition distincte de la première comprenant un milieu cosmétiquement acceptable.

Un autre objet de la présente invention est un produit cosmétique destiné à être appliqué sur les phanères, notamment les ongles, les cheveux ou les sourcils, comprenant une première et une seconde compositions, la première composition contenant, dans un milieu liquide organique cosmétiquement acceptable, au moins un polymère éthylénique greffé, et la seconde composition distincte de la première comprenant un milieu cosmétiquement acceptable.

Le produit de l'invention est en particulier un produit de maquillage de la peau, des ongles ou des cheveux.

Par produit de maquillage, on entend un produit contenant un agent colorant permettant le dépôt d'une couleur sur une matière kératinique (la peau ou les phanères) de personne humaine par l'application sur la matière kératinique de produits tels que des rouges à lèvres, des fards, des eye-liners, des fonds de teint ou des autobronzants, des produits de maquillage semi permanent (tatouage).

Le produit selon l'invention comprend au moins deux compositions cosmétiquement acceptables conditionnées séparément ou ensemble dans un même article de conditionnement ou dans au moins deux articles de conditionnement séparés.

De préférence, ces compositions sont conditionnées séparément et avantageusement, dans des articles de conditionnement séparés.

L'objet de la présente invention est donc en particulier un produit cosmétique de maquillage se présentant sous la forme d'un rouge à lèvres, d'un fond de teint, d'un mascara, d'un fard à joues ou à paupières, d'un rouge à lèvres, d'un vernis à ongle, d'un produit ayant notamment des propriétés de soin, d'un mascara, d'un eye-liner, d'un produit anti-cernes, ou d'un produit de maquillage du corps (du type tatouage) ou des cheveux.

L'invention a aussi pour objet un kit de maquillage contenant un produit cosmétique de maquillage tel que défini précédemment, dans lequel les différentes compositions sont conditionnées séparément et sont avantageusement accompagnées de moyens d'application appropriés. Ces moyens peuvent être des pinceaux, des brosses, des stylos, des crayons, des feutres, des plumes, des éponges, des tubes et /ou des embouts mousse.

La première composition du produit selon l'invention peut constituer une couche de base appliquée sur la matière kératinique et la seconde composition une couche de dessus. Il est toutefois possible d'appliquer sous la première couche une sous couche ayant la constitution ou non de la seconde couche.

Il est aussi possible de déposer une surcouche sur la seconde couche ayant une constitution identique ou non à celle de la première couche. De préférence, le maquillage obtenu est un maquillage bicouche.

La deuxième composition peut également constituer une couche de base appliquée sur la matière kératinique et la première composition une couche de dessus.

En particulier la couche de base est un rouge à lèvres, un fond de teint, un mascara, un brillant à lèvres, un eye liner, un vernis à ongles, un produit de soin pour les ongles, un produit de maquillage du corps, et la couche du dessus (ou « top coat ») un produit de soin
ou de protection.

L'invention se rapporte aussi à un procédé de maquillage de la peau et/ou des lèvres et/ou des phanères consistant à appliquer sur la peau et/ou les lèvres et/ou les phanères un produit cosmétique tel que défini précédemment.

L'invention a encore pour objet un procédé cosmétique de soin ou de maquillage de la peau et/ou des lèvres et/ou des phanères d'être humain, consistant à appliquer sur la peau, les lèvres et/ou les phanères une première couche d'une première composition comprenant une dispersion, dans une phase grasse liquide, de particules d'un polymère éthylénique greffé, puis à appliquer, sur tout ou partie de la première couche, une seconde couche d'une seconde composition comprenant un milieu cosmétiquement acceptable.

Ce procédé peut consister à appliquer sur la peau, les lèvres et/ou les phanères d'être humain une première couche d'une première composition comprenant une dispersion, dans une phase grasse liquide, de particules d'un polymère éthylénique greffé, à laisser sécher ladite première couche, puis à appliquer, sur tout ou partie de la première couche, une seconde couche d'une seconde composition comprenant un milieu cosmétiquement acceptable.

Ce procédé peut également consister à appliquer sur la peau, les lèvres et/ou les phanères d'être humain une première couche d'une composition comprenant un milieu cosmétiquement acceptable, à laisser sécher ladite première couche, puis à appliquer, sur tout ou partie de la première couche, une seconde couche d'une composition comprenant une dispersion, dans une phase grasse liquide, de particules d'un polymère éthylénique greffé.

Le produit selon l'invention peut être appliqué sur la peau aussi bien du visage que du cuir chevelu et du corps, des lèvres, de l'intérieur des paupières inférieures, et des phanères comme les ongles, les cils, les cheveux, les sourcils, voire les poils. La seconde composition peut former des motifs et peut être appliquée avec un stylo, crayon ou tout autre instrument (éponge, doigt, pinceau, brosse, plume). Ce maquillage peut aussi être appliqué sur les accessoires de maquillage comme les faux ongles, faux cils, perruques ou encore des pastilles ou des patchs adhérents sur la peau ou les lèvres (du type mouches).

L'invention a aussi pour objet un support maquillé comprenant une première couche d'une première composition comprenant une dispersion, dans une phase grasse liquide, de particules d'un polymère éthylénique greffé, et une seconde couche d'une seconde composition comprenant un milieu cosmétiquement acceptable, ladite première couche étant appliquée sur le support en premier ou au dessus de tout ou partie de la deuxième couche.

Ce support peut être en particulier un postiche tel qu'une perruque, des faux ongles, des faux cils, ou encore des patchs adhérents sur la peau ou les lèvres (du type mouches).

L'invention se rapporte également à l'utilisation cosmétique du produit cosmétique défini ci-dessus pour améliorer les propriétés de confort, en particulier de non collant et/ou de non dessèchement, et/ou de brillance et/ou de transfert et/ou de migration et/ou de tenue du maquillage sur la peau et/ou les lèvres et/ou les phanères.

L'invention a enfin pour objet l'utilisation d'un produit cosmétique comprenant une première et une seconde compositions, la première composition comprenant une dispersion, dans une phase grasse liquide, de particules d'un polymère éthylénique greffé tel que décrit ci-après, et la seconde composition comprenant un milieu cosmétiquement acceptable, pour conférer à la peau et/ou les lèvres et/ou les phanères un rendu cosmétique confortable et/ou brillant et/ou non transfert et/ou non migrant et /ou de bonne tenue.

Première composition

La première composition selon l'invention comprend une dispersion de particules, de préférence solides, d'un polymère éthylénique greffé dans une phase grasse liquide.

Par polymère "éthylénique", on entend un polymère obtenu par polymérisation de monomères comprenant une insaturation éthylénique.

La dispersion de polymère éthylénique greffé est notamment exempte de polymère stabilisant distinct dudit polymère greffé, tels que ceux décrits dans EP749747, et les particules de polymère éthylénique greffé ne sont donc pas stabilisées en surface par de tels polymères stabilisants additionnels. Le polymère greffé est donc dispersé dans la phase grasse liquide en l'absence de stabilisant additionnel en surface des particules de polymère greffé.

Par polymère greffé, on entend un polymère ayant un squelette comprenant au moins une chaîne latérale pendante ou située en bout de chaîne, et de préférence pendante.

Avantageusement, le polymère éthylénique greffé comprend un squelette éthylénique insoluble dans ladite phase grasse liquide, et des chaînes latérales liées de manière covalente audit squelette et solubles dans ladite phase grasse liquide.

Le polymère éthylénique greffé est notamment un polymère non réticulé. En particulier, le polymère est obtenu par polymérisation de monomères comprenant un seul groupement polymérisable.

De préférence, le polymère éthylénique greffé est un polymère filmogène.
Par polymère "filmogène", on entend un polymère apte à former à lui seul ou en présence d'un agent auxiliaire de filmification, un film continu et adhérent sur un support, notamment sur les matières kératiniques, et de préférence un film cohésif et mieux encore, un film dont la cohésion et les propriétés mécaniques sont telles que ledit film peut être isolé dudit support
Selon un mode de réalisation de l'invention, le polymère éthylénique greffé est un polymère acrylique greffé.
Le polymère éthylénique greffé est notamment susceptible d'être obtenu par polymérisation radicalaire dans un milieu organique de polymérisation :

- d'au moins un monomère éthylénique, en particulier d'au moins un monomère acrylique et éventuellement d'au moins un monomère additionnel vinylique non acrylique, pour former ledit squelette insoluble ; et
- d'au moins un macromonomère comportant un groupe terminal polymérisable pour former les chaînes latérales, ledit macromonomère ayant une masse moléculaire moyenne en poids supérieure ou égale à 200 et la teneur en macromonomère polymérisé représentant de 0,05 à 20 % en poids du polymère.

La phase grasse liquide peut contenir le milieu organique de polymérisation.
Le milieu organique liquide de dispersion, correspondant au milieu dans lequel est fourni le polymère greffé, peut être identique au milieu de polymérisation.
Toutefois, le milieu de polymérisation peut être substitué en tout ou partie par un autre milieu organique liquide. Cet autre milieu organique liquide peut être ajouté, après polymérisation, au milieu de polymérisation. Ce dernier est ensuite évaporé en tout ou partie.
La phase grasse liquide peut contenir des composés liquides organiques autres que ceux présents dans le milieu de dispersion. Ces autres composés sont choisis de manière à ce que le polymère greffé reste à l'état de dispersion dans la phase grasse liquide.
Le milieu liquide organique de dispersion est présent dans la phase grasse liquide de la première composition selon l'invention du fait de l'introduction dans la première composition de la dispersion de polymère greffé obtenue.
La phase grasse liquide comprend, de préférence majoritairement un ou plusieurs composés organiques liquides (ou huiles) tels que définis ci-après.
En particulier, la phase grasse liquide est une phase organique liquide non aqueuse et non miscible à l'eau à la température ambiante (25 °C).
On entend par "composé organique liquide" un composé non aqueux qui est à l'état liquide à la température ambiante (25 °C) et qui s'écoule donc de son propre poids.
On entend par "composé siliconé" un composé contenant au moins un atome de silicium.
Parmi les composés organiques liquides ou huiles pouvant être présents dans le milieu organique liquide de dispersion, on peut citer :

- les composés organiques liquides, notamment non siliconés ou siliconés, ayant un paramètre de solubilité global selon l'espace de solubilité de Hansen inférieur ou égal à 18 $(MPa)^{1/2}$, de préférence inférieur ou égal à 17 $(MPa)^{1/2}$,
- les monoalcools ayant un paramètre de solubilité global selon l'espace de solubilité de Hansen inférieur ou égal à 20 $(MPa)^{1/2}$; et
- leurs mélanges.

Le paramètre de solubilité global δ selon l'espace de solubilité de Hansen est défini dans l'article « Solubility parameter values » de Eric A.Grulke de l'ouvrage « Polymer Handbook », 3éme édition, Chapitre VII, p.519-559 par la relation :

$$\delta = (d_D^{\ 2} + d_P^{\ 2} + d_H^{\ 2})^{1/2}$$

dans laquelle

- $d_D$ caractérise les forces de dispersion de LONDON issues de la formation de dipôles induits lors des chocs moléculaires,
- $d_P$ caractérise les forces d'intéractions de DEBYE entre dipôles permanents, et
- $d_H$ caractérise les forces d'intéractions spécifiques (type liaisons hydrogène, acide/base, donneur/accepteur, etc.).

La définition des solvants dans l'espace de solubilité selon Hansen est décrite dans l'article de C.M.Hansen « The three dimensional solubility parameters » J.Paint Technol. 39, 105 (1967).

Parmi les composés liquides organiques, notamment non siliconés ou siliconés, ayant un paramètre de solubilité global selon l'espace de solubilité de Hansen inférieur ou égal à 18 $(Mpa)^{1/2}$, on peut citer des corps gras liquides, notamment des huiles, qui peuvent être choisis parmi les huiles naturelles ou synthétiques, carbonées, hydrocarbonées, fluorées, siliconées, éventuellement ramifiées, seules ou en mélange.

Par "huile", on entend tout milieu non aqueux liquide à température ambiante (25°C) et pression atmosphérique (760mm de Hg), compatible avec une application sur la peau, les muqueuses (lèvres) et/ou les phanères (ongles, cils, sourcils, cheveux).

Parmi ces huiles, on peut citer les huiles végétales formées par des esters d'acides gras et de polyols, en particulier les triglycérides, telles que l'huile de tournesol, de sésame ou de colza, ou les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates et les benzoates, notamment l'adipate de diisopropyle.

On peut également citer les alcanes linéaires, ramifiés et/ou cycliques éventuellement volatils et notamment des huiles de paraffine, de vaseline, ou le polyisobutylène hydrogéné, l'isododécane, ou encore les 'ISOPARS', les isoparaffines volatiles. On peut citer également les esters, les éthers, les cétones.

On peut encore citer les huiles siliconées telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyles, thiols et/ou amines, et les huiles siliconées volatiles, notamment cycliques.

En particulier, la première composition peut comprendre une huile volatile.

Par huile volatile, on entend tout milieu non aqueux susceptible de s'évaporer de la peau ou des lèvres, en moins d'une heure, ayant notamment une pression de vapeur, à température ambiante et pression atmosphérique allant de $10^{-3}$ à 300 mm de Hg (0,13 Pa à 40 000 Pa).

Avantageusement, la première composition selon l'invention peut comprendre une huile volatile en une teneur allant de 1 % à 70 % en poids, par rapport au poids total de la première composition, de préférence allant de 5 % à 50 % en poids, et préférentiellement allant de 10 % à 35 % en poids.

Comme huile siliconée volatile utilisable dans l'invention, on peut citer les silicones linéaires

ou cycliques ayant de 2 à 7 atomes de silicium, ces silicones comportant éventuellement des groupes alkyle ou alkoxy ayant de 1 à 10 atomes de carbone. En particulier, on peut citer l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.

Comme huile non siliconée volatile, on peut citer l'isododécane, ou encore les 'ISOPARS', les isoparaffines volatiles, l'isohexadécane, l'isodécane.

La première composition peut comprendre une huile non volatile, notamment en une teneur allant de 1 % à 80 % en poids, par rapport au poids total de la première composition, de préférence allant de 5 % à 60 % en poids, et préférentiellement allant de 10 % à 50 % en poids.

Comme huile siliconée non volatile, on peut citer les polydialkylsiloxanes non volatils, tels que les polydiméthylsiloxanes (PDMS) non volatils; les polydiméthylsiloxanes comportant des groupements alkyle, alcoxy ou phényle, pendant ou en bout de chaîne siliconée, groupements ayant de 2 à 24 atomes de carbone; les silicones phénylées comme les phényl triméthicones, les phényl diméthicones, les phényl triméthylsiloxy diphénylsiloxanes, les diphényl diméthicones, les diphényl méthyldiphényl trisiloxanes, les polyméthylphénylsiloxanes; les polysiloxanes modifiés par des acides gras (notamment en $C_8$-$C_{20}$), des alcools gras (notamment en $C_8$-$C_{20}$) ou des polyoxyalkylènes (notamment polyoxyéthyléne et/ou polyoxypropylène); les polysiloxanes aminées ; les polysiloxanes à groupement hydroxyles; les polysiloxanes fluorés comportant un groupement fluoré pendant ou en bout de chaîne siliconée ayant de 1 à 12 atomes de carbone dont tout ou partie des hydrogène sont substitués par des atomes de fluor ; et leurs mélanges.

On peut citer, en particulier, comme composés organiques liquides non siliconés ayant un paramètre de solubilité global selon l'espace de solubilité de Hansen inférieur ou égal à 18 $(Mpa)^{1/2}$:

- les esters linéaires, ramifiés ou cycliques, ayant au moins 6 atomes de carbone, notamment de 6 à 30 atomes de carbone;
- les éthers ayant au moins 6 atomes de carbone, notamment de 6 à 30 atomes de carbone ; et
- les cétones ayant au moins 6 atomes de carbone, notamment de 6 à 30 atomes de carbone.

Par monoalcools liquides ayant un paramètre de solubilité global selon l'espace de solubilité de Hansen inférieur ou égal à 20 $(MPa)^{1/2}$, on entend les monoalcools liquides gras aliphatiques ayant de 6 à 30 atomes de carbone, la chaîne hydrocarbonée ne comportant pas de groupement de substitution. Comme monoalcools selon l'invention, on peut citer l'alcool oléique, le décanol et l'alcool linoléique.

Selon un premier mode de réalisation de l'invention, la phase grasse liquide peut être une phase grasse liquide non siliconée.
On entend par "phase grasse liquide non siliconée" une phase grasse comprenant un ou plusieurs composés organiques liquides ou huiles non siliconé(e)s, tels que ceux cités précédemment, lesdits composés non siliconés étant présents majoritairement dans la phase grasse liquide, c'est-à-dire à au moins 50 % en poids, notamment de 50 à 100 % en poids, de préférence de 60 % à 100 % en poids (par exemple de 60 à 99 % en poids), ou encore de 65 % à 100 % en poids (par exemple de 65 à 95 % en poids), par rapport au poids total de la phase grasse liquide.
Les composés organiques liquides non siliconés peuvent être notamment choisis parmi :

- les composés organiques liquides non s iliconés ayant un paramètre de solubilité global selon l'espace de solubilité de Hansen inférieur ou égal à 18 $(MPa)^{1/2}$,
- les monoalcools ayant un paramètre de solubilité global selon l'espace de solubilité de Hansen inférieur ou égal à 20 $(MPa)^{1/2}$; et
- leurs mélanges.

Ladite phase grasse liquide non siliconée peut donc éventuellement comprendre des composés organiques liquide ou huiles siliconé(e)s, tels que ceux cités précédemment, qui peuvent être présents en une quantité inférieure à 50 % en poids, notamment allant de 0,1 à 40 % en poids, voire allant de 1 à 35 % en poids, ou encore allant de 5 à 30 % en poids, par rapport au poids total de la phase grasse liquide.
Selon un mode particulier de réalisation de l'invention, la phase grasse liquide non siliconée ne contient pas de composés organiques liquides ou huiles siliconé(e)s.
Lorsque la phase grasse liquide est une phase grasse liquide non siliconée, les macromonomères présents dans le polymère greffé sont avantageusement des macromonomères carbonés tels que décrits ci-après.
En particulier, lorsque la phase grasse liquide est une phase grasse liquide non siliconée, le polymère greffé présent dans la première composition est avantageusement un polymère greffé non siliconé.
Par polymère greffé non siliconé, on entend un polymère greffé contenant majoritairement un macromonomère carboné et contenant éventuellement au plus 7 % en poids du poids total du polymère, de préférence au plus 5 % en poids, voire est exempt, de macromonomère siliconé.
Selon un deuxième mode de réalisation de l'invention, la phase grasse liquide peut être une phase grasse liquide siliconée.
On entend par "phase grasse liquide siliconée" une phase grasse comprenant un ou plusieurs composés organiques liquides siliconés ou huiles siliconées tels que ceux décrits précédemment, lesdits composés siliconés étant présents majoritairement dans la phase grasse liquide, c'est-à-dire à au moins 50 % en poids, notamment de 50 à 100 % en poids, de préférence de 60 % à 100 % en poids (par exemple de 60 à 99 % en poids), ou encore de 65 % à 100 % en poids (par exemple de 65 à 95 % en poids), par rapport au poids total de la phase grasse liquide.
Les composés organiques liquides siliconés peuvent être notamment choisis parmi :

- les composés organiques liquides, notamment non siliconés ou siliconés, ayant un paramètre de solubilité global selon l'espace de solubilité de Hansen inférieur ou égal à 18 $(MPa)^{1/2}$, de préférence inférieur ou égal à 17 $(MPa)^{1/2}$

Ladite phase grasse liquide siliconée peut donc éventuellement comprendre des composés organiques liquides ou huiles non siliconé(e)s, tels que décrits précédemment, qui peuvent être présents en une quantité inférieure à 50 % en poids, notamment allant de 0,1 à 40 % en poids, voire allant de 1 à 35 % en poids, ou encore allant de 5 à 30 % en poids, par rapport au poids total da la phase grasse liquide.
Selon un mode particulier de réalisation de l'invention, la phase grasse liquide siliconée ne contient pas de composés organiques liquides non siliconés.
Lorsque la phase grasse liquide est une phase grasse liquide siliconée, les macromonomères présents dans le polymère greffé sont avantageusement des macromonomère siliconés tels que décrits ci-après.
En particulier, lorsque la phase grasse liquide est une phase grasse liquide siliconée, le polymère greffé présent dans la première composition est avantageusement un polymère greffé siliconé.
Par polymère greffé siliconé, on entend un polymère greffé contenant majoritairement un macromonomère siliconé et contenant éventuellement au plus 7 % en poids du poids total du polymère, de préférence au plus 5 % en poids, voire est exempt, de macromonomère carboné.
Le choix des monomères constituant le squelette du polymère, des macromonomères, le poids moléculaire du polymère, la proportion des monomères et des macromonomères peut être fait en fonction du milieu organique liquide de dispersion de manière à obtenir avantageusement une dispersion de particules de polymères greffés en particulier une dispersion stable, ce choix pouvant être effectué par l'homme du métier.
Par "dispersion stable", on entend une dispersion qui n'est pas susceptible de former de dépôt solide ou de

déphasage liquide/solide notamment après une centrifugation, par exemple, à 4000 tours/minute pendant 15 minutes.

Le polymère éthylénique greffé formant les particules en dispersion comprend donc un squelette insoluble dans ledit milieu de dispersion et une partie soluble dans ledit milieu de dispersion.

Le polymère éthylénique greffé peut être un polymère statistique.

Selon l'invention, on entend par "polymère éthylénique greffé " un polymère susceptible d'être obtenu par polymérisation radicalaire :

- d'un ou plusieurs monomère(s) éthylénique(s),
- avec un ou plusieurs macromonomère(s), dans un milieu organique de polymérisation.

Selon l'invention, on entend par "polymère acrylique greffé " un polymère susceptible d'être obtenu par polymérisation radicalaire :

- d'un ou plusieurs monomère(s) acrylique(s), et éventuellement d'un ou plusieurs monomère(s) additionnel(s) vinylique(s) non acrylique(s),
- avec un ou plusieurs macromonomère(s), dans un milieu organique de polymérisation.

Avantageusement, les monomères acryliques représentent de 50 à 100 % en poids, de préférence de 55 à 100 % en poids (notamment de 55 à 95 % en poids), préférentiellement de 60 à 100 % en poids (notamment de 60 à 90 % en poids) du mélange monomères acryliques + monomères vinyliques non acryliques éventuels.

De préférence, les monomères acryliques sont choisis parmi les monomères dont l'homopolymère est insoluble dans le milieu de dispersion considéré, c'est-à-dire que l'homopolymère est sous forme solide (ou non dissous) à une concentration supérieure ou égale à 5% en poids à température ambiante (20°C) dans ledit milieu de dispersion.

Selon l'invention, on entend par "macromonomère ayant un groupe terminal polymérisable" tout polymère comportant sur une seule de ses extrémités un groupe terminal polymérisable apte à réagir lors de la réaction de polymérisation avec les monomères acryliques et éventuellement les monomères vinyliques non acryliques additionnels constituant le squelette. Le macromonomère permet de former les chaînes latérales du polymère acrylique greffé. Le groupe polymérisable du macromonomère peut être avantageusement un groupe à insaturation éthylénique susceptible de se polymériser par voie radicalaire avec les monomères constituant le squelette.

Par "macromonomère carboné" on entend un macromonomère non siliconé, et notamment un macromonomère oligomère obtenu par polymérisation de monomère(s) non siliconé(s) à insaturation éthylénique, et principalement par polymérisation de monomères acryliques et/ou vinyliques non acryliques.

Par "macromonomère siliconé" on entend un macromonomère organopolysiloxane, et en particulier un macromonomère polydiméthylsiloxane.

De préférence, le macromonomère est choisi parmi les macromonomères dont l'homopolymère est soluble dans le milieu de dispersion considéré, c'est-à-dire complètement dissous à une concentration supérieure ou égale à 5 % en poids et à température ambiante dans ledit milieu de dispersion.

Ainsi, le polymère acrylique greffé comprend un squelette (ou chaîne principale) constitué par un enchaînement de motifs acryliques résultant de la polymérisation notamment d'un ou plusieurs monomères acryliques et des chaînes latérales (ou greffons) issus de la réaction des macromonomères, lesdites chaînes latérales étant liées de manière covalente à ladite chaîne principale.

Le squelette (ou chaîne principale) est insoluble dans le milieu de dispersionconsidéré alors que les chaînes latérales (ou greffons) sont solubles dans ledit milieu de dispersion.

Par "monomère acryliques", on entend dans la présente demande des monomères choisis parmi l'acide (méth)acrylique, les esters de l'acide (méth)acrylique (appelés également les (méth)acrylates), les amides de l'acide (méthacrylique) (appelés également les (méth)acrylamides).

Comme monomère acrylique susceptible d'être employé pour former le squelette insoluble du polymère, on peut citer, seul ou en mélange, les monomères suivants, ainsi que leurs sels :

- (i) les (méth)acrylates de formule :

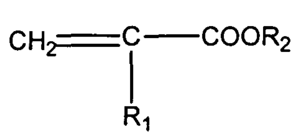

dans laquelle :

- $R_1$ désigne un atome d'hydrogène ou un groupe méthyle ;
- $R_2$ représente un groupe choisi parmi :

  - un groupe alkyle linéaire ou ramifié, comprenant de 1 à 6 atomes de carbone, ledit groupe pouvant comporter dans sa chaîne un ou plusieurs hétéroatomes choisis parmi O, N et S; et/ou pouvant comporter un ou plusieurs substituants choisis parmi
  - OH, les a tomes d'hatogène (F, Cl, Br, I) et -NR'R" avec R' et R" identiques ou différents choisis parmi les alkyles linéaires ou ramifiés en $C_1$-$C_4$; et/ou pouvant être substitué par au moins un groupe polyoxyalkylène, en particulier avec alkylène en $C_2$-$C_4$, notamment polyoxyéthylène et/ou polyoxypropylène, ledit groupe polyoxyalkylène étant constitué par la répétition de 5 à 30 motifs oxyalkylène;
  - un groupe alkyle cyclique comprenant de 3 à 6 atomes de carbone, ledit groupe pouvant comporter dans sa chaîne un ou plusieurs hétéroatomes choisis parmi O, N et S, et/ou pouvant comporter un ou plusieurs substituants choisis parmi OH et les atomes d'halogène (F, Cl, Br, I);

A titre d'exemples de $R_2$, on peut citer le groupe méthyle, éthyle, propyle, butyle, isobutyle, méthoxyéthyle, éthoxyéthyle, méthoxy-polyoxyéthylène 350 OE , trifluoroéthyle, 2-hydroxyéthyle, 2-hydroxypropyle, diméthylaminoéthyle, diéthylaminoéthyle, diméthylaminopropyle.

- (ii) les (méth)acrylamides de formule :

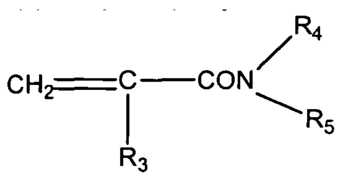

dans laquelle :

- $R_3$ désigne un atome d'hydrogène ou un groupe méthyle ;
- $R_4$ et $R_5$, identiques ou différents, représentent un atome d'hydrogène ou un groupe alkyle, linéaire ou ramifié, comportant de 1 à 6 atomes de carbone, pouvant comporter un ou plusieurs substituants choisis parmi -OH, les atomes d'halogène (F, Cl, Br, I) et -NR'R" avec R' et R" identiques ou différents choisis parmi les alkyles linéaires ou ramifiés en $C_1$-$C_4$;ou
- $R_4$ représente un atome d'hydrogène et $R_5$ représente un groupe 1,1-diméthyl-3-oxobutyle. A titre d'exemples de groupes alkyles pouvant constituer $R_4$ et $R_5$, on peut citer n-butyle, t-butyle, n-propyle, diméthylaminoéthyle, diéthylaminoéthyle, diméthylaminopropyle.

- (iii) les monomères (méth)acryliques comprenant au moins une fonction acide carboxylique, phosphorique ou sulfonique, tels que l'acide acrylique, l'acide méthacrylique, l'acide acrylamidopropanesulfonique.

Parmi ces monomères acryliques, on peut tout particulièrement citer les (méth)acrylates de méthyle, d'éthyle, de propyle, de butyle, d'isobutyle; les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle ; le diméthylaminopropylméthacrylamide; l'acide (méth)acrylique ; et leurs sels ; et leurs mélanges.
De préférence, les monomères acryliques sont choisis parmi l'acrylate de méthyle, l'acrylate de méthoxyéthyle, le méthacrylate de méthyle, le méthacrylate de 2-hydroxyéthyle, l'acide (méth)acrylique, le méthacrylate de diméthylaminoéthyle, et leurs mélanges.
Parmi les monomères additionnels vinyliques non acryliques, on peut citer :

- les esters vinylique de formule : $R_6$-COO-CH=$CH_2$

dans laquelle $R_6$ représente un groupe alkyle linéaire ou ramifié, comprenant de 1 à 6 atomes, ou un groupe alkyle cyclique comportant de 3 à 6 atomes de carbone et/ou un groupe aromatique, par exemple de type benzénique, anthracénique, et naphtalénique ;

- les monomères vinyliques non acryliques comprenant au moins une fonction acide carboxylique, phosphorique

ou sulfonique, tels que l'acide crotonique, l'anhydride maléique, l'acide itaconique, l'acide fumarique, l'acide maléique, l'acide styrènesulfonique, l'acide vinylbenzoïque, l'acide vinylphosphorique, et leurs sels ;

- les monomères vinyliques non acryliques comprenant au moins une fonction amine tertiaire, tels que la 2-vinylpyridine, la 4-vinylpyridine ;
- et leurs mélanges.

Selon un mode de réalisation de l'invention, le polymère greffé comprend de l'acide (méth)acrylique.
Avantageusement, les monomères acryliques présents dans le polymère greffé comprennent au moins l'acide (méth)acrylique, et en particulier au moins l'acide (méth)acrylique et au moins un monomère choisi parmi les (méth) acrylates et les (méth)acrylamides décrits précédemment aux points (i) et (ii). De préférence, les monomères acryliques comprennent au moins l'acide (méth)acrylique et au moins un monomère choisi parmi les (méth)acrylates d'alkyle en $C_1$-$C_3$. L'acide (méth)acrylique peut être présent en une teneur d'au moins 5 % en poids, par rapport au poids total du polymère (notamment allant de 5 % à 80 % en poids), de préférence d'au moins 10 % en poids (notamment allant de 10 % en poids à 70 % en poids), préférentiellement d'au moins 15 % en poids (notamment allant de 15 % à 60 % en poids).
Parmi les sels, on peut citer ceux obtenus par neutralisation des groupements acides à l'aide de base inorganiques telles que l'hydroxyde de sodium, l'hydroxyde de potassium l'hydroxyde d'ammonium ou de bases organiques de type alkanols amines comme la monoéthanolamine, la diéthanolamine, la triéthanolamine, la 2-méthyl-2-amino-1-propanol. On peut également citer les sels formés par neutralisation des motifs amine tertiaire, par exemple à l'aide d'acide minéral ou organique. Parmi les acides minéraux, on peut citer l'acide sulfurique ou l'acide chlorhydrique, l'acide bromhydrique, iodhydrique, l'acide phosphorique, l'acide borique. Parmi les acides organiques, on peut citer les acides comportant un ou plusieurs groupes carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles. On peut notamment citer l'acide acétique ou l'acide propionique, l'acide téréphtalique, ainsi que l'acide citrique et l'acide tartrique.
Selon un mode de réalisation de l'invention, le polymère éthylénique greffé ne contient pas de monomères vinyliques non acryliques additionnels tels que décrits précédemment. Dans ce mode de réalisation, le squelette insoluble du polymère éthylénique greffé est formé uniquement de monomères acryliques tels que décrits précédemment.
Il est entendu que ces monomères acryliques non polymérisés peuvent être solubles dans le milieu de dispersion considéré, mais le polymère formé avec ces monomères est insoluble dans le milieu de dispersion.
Selon un mode particulier de réalisation de l'invention, le polymère éthylénique greffé est susceptible d'être obtenu par polymérisation radicalaire dans un milieu organique de polymérisation :

- d'un monomère acrylique principal choisi parmi les (méth)acrylates d'alkyle en $C_1$-$C_3$, seul ou en mélange, et éventuellement d'un ou plusieurs monomères acryliques additionnels choisis parmi l'acide acrylique, l'acide méthacrylique et les (méth)acrylates d'alkyle de formule (I) définie ci-après, et leurs sels, pour former ledit squelette insoluble ; et
- et d'au moins un macromonomère siliconé comportant un groupe terminal polymérisable, tel que défini précédemment.

Comme monomère acrylique principal, on peut utiliser l'acrylate de méthyle, le méthacrylate de méthyle, l'acrylate d'éthyle, le méthacrylate d'éthyle, l'acrylate de n-propyle, le méthacrylate de n-propyle, l'acrylate d'iso-propyle et le méthacrylate d'iso-propyle, et leurs mélanges.
On préfère tout particulièrement l'acrylate de méthyle, le méthacrylate de méthyle, le méthacrylate d'éthyle.
Les monomères acryliques additionnels peuvent être choisis parmi :

- l'acide (méth)acrylique et ses sels,
- les (méth)acrylates de formule (I) et leurs sels :

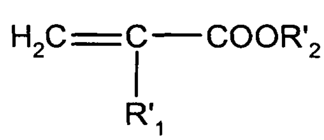

(I)

dans laquelle :

- $R'_1$ désigne un atome d'hydrogène ou un groupe méthyle ;
- $R'_2$ représente

  - un groupe alkyle linéaire ou ramifié comprenant de 1 à 6 atomes de carbone, ledit groupe comportant dans sa chaîne un ou plusieurs atomes d'oxygène et/ou comportant un ou plusieurs substituants choisis parmi
    -OH, les atomes d'halogène (F, Cl, Br, I) et -NR'R" avec R' et R" identiques ou différents choisis parmi les alkyles linéaires ou ramifiés en $C_1$-$C_3$ ;

    - un groupe alkyle cyclique comprenant de 3 à 6 atomes de carbone, ledit groupe pouvant comporter dans sa chaîne un ou plusieurs atomes d'oxygène et/ou pouvant comporter un ou plusieurs substituants choisis parmi OH et les atomes d'halogène (F, Cl, Br, I) ;

- et leurs mélanges.

A titre d'exemples de $R'_2$, on peut citer le groupe méthoxyéthyle, éthoxyéthyle, trifluoroéthyle; 2-hydroxyéthyle, 2-hydroxypropyle, diméthylaminoéthyle, diéthylaminoéthyle, diméthylaminopropyle.
Parmi ces monomères acryliques additionnels, on peut tout particulièrement citer l'acide (méth)acrylique, les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle, leurs sels, et leurs mélanges.
On préfère tout particulièrement l'acide acrylique, l'acide méthylacrylique.
Les macromonomères comportent à une des extrémités de la chaîne un groupe terminal polymérisable apte à réagir au cours de la polymérisation avec les monomères acryliques et éventuellement les monomères vinyliques additionnels, pour former les chaînes latérales du polymère éthylénique greffé. Ledit groupe terminal polymérisable peut être en particulier un groupe vinyle ou (méth)acrylate (ou (méth)acrylol), et de préférence un groupe (méth)acrylate.
Les macromonomères sont choisis préférentiellement parmi les macromonomères dont l'homopolymère a une température de transition vitreuse (Tg) inférieure ou égale à 25°C, notamment allant de - 100°C à 25°C, de préférence allant de
- 80°C à 0°C.
Les macromonomères ont une masse moléculaire moyenne en poids supérieure ou égale à 200, de préférence supérieure ou égale à 300, préférentiellement supérieure ou égale à 500, et plus préférentiellement supérieure à 600.
De préférence, les macromonomères ont une masse moléculaire moyenne en poids (Mw) allant de 200 à 100 000, de préférence allant de 500 à 50 000, préférentiellement allant de 800 à 20 000, plus préférentiellement allant de 800 à 10000, et encore plus préférentiellement allant de 800 à 6000.
Dans la présente demande, les masses molaires moyennes en poids (Mw) et en nombre (Mn) sont déterminées par chromatographie liquide par perméation de gel (solvant THF, courbe d'étalonnage établie avec des étalons de polystyrène linéaire, détecteur réfractométrique).
Comme macromonomères carbonés, on peut en particulier citer :

- (i) les homopolymères et les copolymères (méth)acrylate d'alkyle linéaire ou ramifié en C8-C22, présentant un groupe terminal polymérisable choisi parmi les groupes vinyle ou (méth)acrylate parmi lesquels on peut citer en particulier : les macromonomères de poly(acrylate d'éthyl-2 hexyle) à extrémité mono(méth)acrylate ; les macromonomères de poly(acrylate de dodécyle) ou de poly(méthacrylate de dodécyle) à extrémité mono(méth)acrylate ; les macromonomères de poly(acrylate de stéaryle) ou de poly (méthacrylate de stéaryle) à extrémité mono(méth) acrylate.
  De tels macromonomères sont notamment décrits dans les brevets EP895467 et EP96459 et dans l'article Gillman K.F., Polymer Letters, Vol 5, page 477-481 (1967).
  On peut en particulier citer les macromonomères à base de poly(acrylate d'éthyl-2-hexyle) ou de poly(acrylate de dodécyle) à extrémité mono(méth)acrylate.

- (ii) les polyoléfines ayant un groupe terminal à insaturation éthylénique , en particulier ayant un groupement terminal (méth)acrylate. Comme exemple de telles polyoléfines, on peut citer en particulier les macromonomères suivants, étant entendu qu'ils ont un groupe terminal (méth)acrylate : les macromonomères de polyéthylène, les macromonomères de polypropylène, les macromonomères de copolymère polyéthylène/polypropylène, les macromonomères de copolymère polyéthylène/polybutylène, les macromonomères de polyisobutylène ; les macromonomères de polybutadiène; les macromonomères de polyisoprène ; les macromonomères de polybutadiène; les macromonomères de poly(éthylène/butylène)-polyisoprène ;

De tels macromonomères sont en particulier décrits dans US5625005 qui mentionne des macromonomères éthylène/butylène et éthylène/propylène à groupement terminal réactif (méth)acrylate.

On peut en particulier citer le méthacrylate de poly(éthylène/butylène), tel que celui commercialisé sous la dénomination Kraton Liquid L-1253 par Kraton Polymers.

Comme macromonomères siliconés, on peut en particulier citer les polydiméthylsiloxanes à groupement terminal mono (méth)acrylate, et notamment ceux de formule (II) suivante :

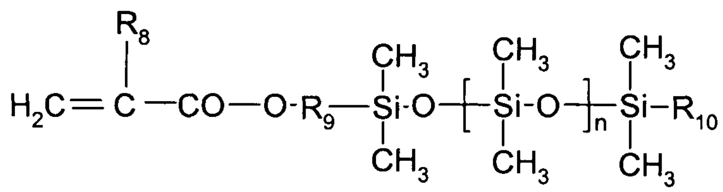

(II)

dans laquelle $R_8$ désigne un atome d'hydrogène ou un groupement méthyle ; $R_9$ désigne un groupe hydrocarboné divalent ayant de 1 à 10 atomes de carbone et contient éventuellement une ou deux liaisons éther -O- ; R10 désigne un groupe alkyl ayant de 1 à 10 atomes de carbone, notamment de 2 à 8 atomes de carbone ; n désigne un nombre entier allant de 1 à 300, de préférence allant de 3 à 200, et préférentiellement allant de 5 à 100.

Comme macromonomères siliconés, on peut utiliser les monométhacryloxypropyl polydiméthylsiloxanes tels que ceux commercialisés sous la dénomination PS560-K6 par la société United Chemical Technologies Inc. (UCT) ou sous la dénomination MCR-M17 par la société Gelest Inc.

De préférence, le macromonomère polymérisé (constituant les chaînes latérales du polymère greffé) représente de 0,1 à 15 % en poids du poids total du polymère, préférentiellement de 0,2 à 10 % en poids, et plus préférentiellement de 0,3 à 8 % en poids.

Comme polymère éthylénique greffé particulièrement préféré dispersé dans une phase grasse liquide non siliconée, on peut utiliser ceux obtenus par polymérisation :

- de l'acrylate de méthyle et du macromonomère polyéthylène/polybutylène à groupement terminal méthacrylate (notamment Kraton L-1253), en particulier dans un solvant choisi parmi l'isododécane, l'isononanoate d'isononyle, l'octyldodécanol, le malate de diisostéaryle, un benzoate d'alkyl $C_{12}$-$C_{15}$ (tel que Finsolv TN) ;
- de l'acrylate de méthoxyéthyle et du macromonomère polyéthylène/polybutylène à groupement terminal méthacrylate (notamment Kraton L-1253), en particulier dans l'isododécane ;
- des monomères acrylate de méthyle/méthacrylate de méthyle et du macromonomère polyéthylène/polybutylène à groupement terminal méthacrylate (notamment Kraton L-1253), en particulier dans l'isododécane ;
- des monomères acrylate de méthyle / acide acrylique et du macromonomère polyéthylène/polybutylène à groupement terminal méthacrylate (notamment Kraton L-1253), en particulier dans l'isododécane ;
- des monomères acrylate de méthyle / méthacrylate de diméthylaminoéthyle et du macromonomère polyéthylène/polybutylène à groupement terminal méthacrylate (notamment Kraton L-1253), en particulier dans l'isododécane ;
- des monomères acrylate de méthyle / méthacrylate de 2-hydroxyéthyle et du macromonomère polyéthylène/polybutylène à groupement terminal méthacrylate (notamment Kraton L-1253), en particulier dans l'isododécane.

Comme polymère acrylique greffé particulièrement préféré dispersé dans une phase grasse liquide siliconée, on peut utiliser ceux obtenus par polymérisation :

- de l'acrylate de méthyle et du macromonomère monométhacryloxypropylpolydiméthylsiloxane ayant un poids moléculaire moyen en poids allant de 800 à 6000, en particulier dans le décaméthylcyclopentasiloxane ou le phényltriméthicone ;
- de l'acrylate de méthyle, d'acide acrylique et du macromonomère monométhacryloxypropylpolydiméthylsiloxane ayant un poids moléculaire moyen en poids allant de 800 à 6000, en particulier dans le décaméthylcyclopentasiloxane ou le phényltriméthicone.

De préférence, le polymère greffé a une masse moléculaire moyenne en poids (Mw) comprise entre 10 000 et 300 000, notamment entre 20 000 et 200 000, mieux encore entre 25 000 et 150 000.

Grâce aux caractéristiques susmentionnées, dans un milieu organique de dispersion donné, les polymères ont la capacité de se replier sur eux-mêmes, formant ainsi des particules de forme sensiblement sphérique, avec sur le pourtour de ces particules les chaînes latérales déployées, qui assurent la stabilité de ces particules. De telles particules résultant des caractéristiques du polymère greffé ont la particularité de ne pas s'agglomérer dans ledit milieu et donc de s'autostabiliser et de former une dispersion de particules de polymère particulièrement stable.
En particulier, les polymères éthyléniques greffés de la dispersion peuvent former des particules nanométriques, de taille moyenne allant de 10 à 400 nm, de préférence de 20 à 200 nm.
Du fait de cette taille très faible, les particules de polymère greffé en dispersion sont particulièrement stables et donc peu susceptibles de former des agglomérats.
La dispersion de polymère greffé peut donc être une dispersion stable et ne forme pas de sédiments, lorsqu'elle est placée pendant une durée prolongée (par exemple 24 heures) à température ambiante (25 °C).

De préférence, la dispersion de particules de polymère greffé présente un taux de matière sèche (ou extrait sec) en polymère pouvant aller de 40 % à 70 % en poids de matière sèche, notamment allant de 45 % à 65 % en poids.

On peut préparer la dispersion de particules de polymère greffé par un procédé comprenant une étape de copolymérisation radicalaire, dans un milieu organique de polymérisation, d'un ou plusieurs monomères acryliques tels que définis précédemment avec un ou plusieurs macromonomères tels que définis précédemment.
Comme indiqué précédemment, le milieu organique liquide de dispersion peut être identique ou différent du milieu de polymérisation.

D'une manière classique, la copolymérisation peut être effectuée en présence d'un initiateur de polymérisation. Les initiateurs de polymérisation peuvent être des amorceurs radicalaires. De manière générale, un tel initiateur de polymérisation peut être choisi parmi les composés organiques peroxydés tels que le dilauroyl peroxyde, le dibenzoyl peroxyde, le tert-butyl peroxy-2-éthylhexanoate ; les composés diazotés tels que l'azobisisobutyronitrile, l'azobisdiméthylvalero-nitrile.
La réaction peut être également initiée à l'aide de photoinitiateurs ou par une radiation telle que des UV, des neutrons ou par plasma.
D'une manière générale, pour mettre en oeuvre ce procédé, on introduit, dans un réacteur de taille appropriée à la quantité de polymère que l'on va réaliser, au moins une partie du milieu organique de polymérisation, une partie des monomères acryliques et/ou vinyliques additionnels, qui constituera, après polymérisation, le squelette insoluble, la totalité du macromonomère (qui constituera les chaînes latérales du polymère) et une partie de l'initiateur de polymérisation. A ce stade d'introduction, le milieu réactionnel forme un milieu relativement homogène.
Le milieu réactionnel est ensuite agité et chauffé jusqu'à une température pour obtenir une polymérisation des monomères et macromonomères. Après un certain temps, le milieu initialement homogène et limpide conduit à une dispersion d'aspect laiteux. On ajoute ensuite un mélange constitué de la partie restante de monomères et de l'initiateur de polymérisation. Après un temps adéquat pendant lequel le mélange est chauffé sous agitation, le milieu se stabilise sous forme d'une dispersion laiteuse, la dispersion comprenant des particules de polymères stabilisés dans le milieu dans lequel elles ont été créées, ladite stabilisation étant due à la présence, dans le polymère, de chaînes latérales solubles dans ledit milieu de dispersion.

La première composition selon l'invention permet d'obtenir un dépôt, notamment un dépôt de maquillage, sur les matières kératiniques, notamment sur la peau ou les lèvres ayant une bonne brillance et de bonnes propriétés de sans transfert.

La première composition selon l'invention permet également d'obtenir un dépôt sur les matières kératiniques, notamment sur la peau ou les lèvres ne présentant pas de sensation de dessèchement, de tiraillement : le dépôt ainsi obtenu est donc confortable au cours du temps pour l'utilisatrice.

Le polymère greffé décrit précédemment peut être présent dans la première composition selon l'invention en une teneur allant de 0,1 % à 70 % en poids, par rapport au poids total de la première composition, de préférence allant de 0,5 % à 50 % en poids, et préférentiellement allant de 1 % à 40 % en poids.

Les présents exemples illustrent la préparation de polymères conformes à l'invention, aptes à former une dispersion de particules dans un milieu organique considéré.

Dans ces exemples, on détermine, après préparation de ladite dispersion, les masses molaires moyennes en poids (Mw) et en nombre (Mn) du polymère, la température de transition vitreuse du polymère, le taux de matière sèche (ou extrait sec) de la dispersion et la taille des particules de polymères.

Les masses molaires moyennes en poids (Mw) et en nombre (Mn) sont déterminées par chromatographie liquide par perméation de gel (solvant THF, courbe d'étalonnage établie avec des étalons de polystyrène linéaire, détecteur réfractométrique).

La mesure de la température de transition vitreuse (Tg) est effectuée selon la norme ASTM D3418-97, par analyse enthalpique différentielle (DSC " Differential Scanning Calorimetry") sur calorimètre, sur une plage de température comprise entre -100°C et +150°C à une vitesse de chauffe de 10°C/min dans des creusets en aluminium de 150 µl.

La préparation des creusets se fait de la manière suivante : dans un creuset en aluminium de 150 µl on introduit 100 µl de la dispersion obtenue et on laisse le solvant s'évaporer pendant 24h à température ambiante et à 50% d'humidité relative. On renouvelle l'opération puis on introduit le creuset dans le calorimètre Mettler DSC30.

Le taux de matière sèche (ou extrait sec), c'est à dire la teneur en matière non volatile, peut être mesurée de différentes manières: on peut citer par exemple les méthodes par séchage à l'étuve ou les méthodes par séchage par exposition à un rayonnement infrarouge.

De préférence, le taux de matière sèche est mesuré par échauffement de l'échantillon par des rayons infrarouges de 2 µm à 3,5 µm de longueur d'onde. Les substances contenues dans la composition qui possèdent une pression de vapeur élevée s'évaporent sous l'effet de ce rayonnement. La mesure de la perte de poids de l'échantillon permet de déterminer l'extrait sec de la composition. Ces mesures sont réalisées au moyen d'un dessiccateur à infrarouges commercial LP16 de chez Mettler. Cette technique est parfaitement décrite dans la documentation de l'appareil fournie par Mettler.

Le protocole de mesure est le suivant : on étale environ 1g de la composition sur une coupelle métallique. Celle-ci, après introduction dans le dessiccateur, est soumise à une consigne de température de 120°C pendant une heure. La masse humide de l'échantillon, correspondant à la masse initiale et la masse sèche de l'échantillon, correspondant à la masse après exposition au rayonnement, sont mesurées au moyen d'une balance de précision.

Le taux de matière sèche est calculée de la manière suivante :

Extrait Sec = 100 x (masse sèche / masse humide).

Les tailles de particules peuvent être mesurées par différentes techniques : on peut citer en particulier les techniques de diffusion de la lumière (dynamiques et statiques), les méthodes par compteur Coulter, les mesures par vitesse de sédimentation (reliée à la taille via la loi de Stokes) et la microscopie. Ces techniques permettent de mesurer un diamètre de particules et pour certaines d'entre elles une distribution granulométrique.

De préférence, les tailles et les distributions de tailles des particules des compositions selon l'invention, sont mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957.

La composition est caractérisé par son diamètre « effectif » moyen en volume D[4,3], défini de la manière suivante :

$$D[4,3] = \frac{\sum_i V_i \cdot d_i}{\sum_i V_i}$$

où $V_i$ représente le volume des particules de diamètre effectif $d_i$. Ce paramètre est notamment décrit dans la documentation technique du granulomètre.

Les mesures sont réalisées à 25 °C, sur une dispersion de particules diluée, obtenue à partir de la composition de la manière suivante : 1) dilution d'un facteur 100 avec de l'eau, 2) homogénéisation de la solution, 3) repos de la solution durant 18 heures, 4) récupération du surnageant homogène blanchâtre.

Le diamètre « effectif » est obtenu en prenant un indice de réfraction de 1,33 pour l'eau et un indice de réfraction moyen de 1,42 pour les particules.

**Exemple 1 : polymère obtenu par polymérisation d'acrylate de méthyle et du macromonomère correspondant à un copolymère polyéthylène/polybutylène à groupement terminal méthacrylate (Kraton L-1253).**

Dans un réacteur, on charge 2 kg d'heptane, 2 kg d'isododécane, 2,8 kg d'acrylate de méthyle et 1,2 kg de macromonomère du type copolymère de polyéthylène/polybutylène à groupement terminal méthacrylate (Kraton L-1253) et 320 g de tertio butyl peroxy-2-éthylhexanoate (Trigonox 21 S).

On agite et on chauffe le mélange réactionnel à température ambiante à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 16 kg d'acrylate de méthyle et 200 g de Trigonox 21 S.

On laisse ensuite le chauffage pendant 4 heures à 90°C puis on distille l'heptane du milieu réactionnel. A l'issue de cette opération de distillation, on obtient une dispersion de particules de polymère ainsi préparé stable dans l'isododécane

Le polymère greffé comprend 6% en poids de macromonomère par rapport au poids du polymère.

Les caractéristiques du polymère et des particules formées par ledit polymère sont les suivantes :

- Masse moléculaire poids Mw = 119900
- Masse moléculaire nombre Mn = 16300
- Indice de polydispersité (Mw/Mn) = 7.37
- Transition vitreuse : 10°C par DSC Mettler ;
- Extrait sec : 52.4 % dans l'isododécane, réalisé par thermobalance ;
- Granulométrie : 46 nm avec polydispersité de 0,05 réalisée sur Malvern Autosizer Lo-C à 25°C

La stabilité de la dispersion obtenue est mise en évidence par la mise en oeuvre du protocole de stabilité suivant : dans un tube à hémolyse, on place 8 ml de la dispersion réalisée et on centrifuge à 4000 tours/min pendant 15 minutes à l'aide d'une centrifugeuse Jouan C100-S5. Au bout de 15 minutes, on constate qu'il n'y a pas de déphasage ce qui démontre que la dispersion est stable.

La composition selon l'invention peut également comprendre au moins un corps gras solide à température ambiante notamment choisi parmi les cires, les corps gras pâteux, les gommes et leurs mélanges. Ces corps gras peuvent être d'origine animale, végétale, minérale ou synthétique.

Par corps gras pâteux, on entend un composé lipophile comportant à la température de 23°C une fraction liquide et une fraction solide. Par corps gras pâteux, on entend également le polylaurate de vinyle.

Ledit composé pâteux a de préférence une dureté à 20°C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20°C au centre de 5 échantillons. Le cylindre est introduit dans chaque échantillon à une pré-vitesse de 1 mm/s puis à une vitesse de mesure de 0,1 mm/s, la profondeur de pénétration étant de 0,3 mm. La valeur relevée de la dureté est celle du pic maximum.

La fraction liquide du composé pâteux mesurée à 23°C représente de préférence 9 à 97% en poids du composé. Cette fraction liquide à 23°C représente de préférence entre 15 et 85%, de préférence encore entre 40 et 85% en poids. La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999.

L'enthalpie de fusion du composé pâteux est la quantité d'énergie nécessaire pour faire passer le composé de l'état solide à l'état liquide. Elle est exprimée eu J/g.

L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du composé pâteux mesurée à 32°C représente de préférence de 30 à 100% en poids du composé, de préférence de 80 à 100%, de préférence encore de 90 à 100% en poids du composé. Lorsque la fraction liquide du composé pâteux mesurée à 32°C est égale à 100%, la température de la fin de la plage de fusion du composé pâteux est inférieure ou égale à 32°C.

La fraction liquide du composé pâteux mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

Par cire au sens de la présente invention, on entend un composé lipophile, solide à température ambiante (25°C), à changement d'état solide/liquide réversible, ayant un point de fusion supérieur ou égal à 30°C pouvant aller jusqu'à 120°C.

Le point de fusion de la cire peut être mesuré à l'aide d'un calorimètre à balayage différentiel (D.S.C.), par exemple le calorimètre vendu sous la dénomination DSC 30 par la Société METLER.

Les cires peuvent être hydrocarbonées, fluorées et/ou siliconées et être d'origine végétale, minérale, animale et/ou synthétique. En particulier, les cires présentent une température de fusion supérieure à 25°C et mieux supérieure à 45°C.

Comme cire utilisable dans la composition de l'invention, on peut citer la cire d'abeilles la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméthicone ayant de 16 à 45 atomes de carbone.

Les gommes sont généralement des polydiméthylsiloxanes (PDMS) à haut poids moléculaire ou des gommes de cellulose ou des polysaccharides.

La nature et la quantité des corps solides sont fonction des propriétés mécaniques et des textures recherchées. A titre indicatif, la composition peut contenir de 0,1 à 50 % en poids de cires, par rapport au poids total de la composition et mieux de 1 à 30 % en poids.

La composition peut ainsi comprendre de l'eau ou un mélange d'eau et de solvant(s) organique(s) hydrophile (s) comme les alcools et notamment les monoalcools inférieurs linéaires ou ramifiés ayant de 2 à 5 atomes de carbone comme l'éthanol, l''isopropanol ou le n-propanol, et les polyols comme la glycérine, la diglycérine, le propylène glycol, le sorbitol, le penthylène glycol, et les polyéthylène glycols, ou bien encore des éthers en $C_2$ et des aldéhydes en $C_2$—$C_4$ hydrophiles.

L'eau ou le mélange d'eau et de solvants organiques hydrophiles peut être présent dans la composition selon l'invention en une teneur allant de 0,1% à 99 % en poids, par rapport au poids total de la composition, et de préférence de 1% à 80%, et de préférence encore de 10 % à 80 % en poids.

La première composition peut comprendre, outre le polymère greffé, un polymère additionnel tel qu'un polymère filmogène.

Parmi les polymères filmogènes utilisables dans la composition de la présente invention, on peut citer les polymères synthétiques, de type radicalaire ou de type polycondensat, les polymères d'origine naturelle et leurs mélanges.

Le polymère filmogène peut être choisi en particulier parmi les polymères cellulosiques tels que la nitrocellulose, l'acétate de cellulose, l'acétobutyrate de cellulose, l'acétopropionate de cellulose, l'éthyl cellulose, ou bien encore les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines aryl-sulfonamide époxy.

Comme polymère filmogène, on peut notamment utiliser la nitrocellulose RS 1/8 sec ; RS ¼ sec. ; ½ sec. ; RS 5 sec. ; RS 15 sec. ; RS 35 sec. ; RS 75 sec.; RS 150 sec ; AS ¼ sec. ; AS ½ sec. ; SS ¼ sec. ; SS ½ sec. ; SS 5 sec., notamment commercialisée par la société HERCULES ; les résine toluène sulfonamide formaldéhyde "Ketjentflex MS80" de la société AKZO ou "Santolite MHP", "Santolite MS 80" de la société FACONNIER ou "RESIMPOL 80" de la société PAN AMERICANA, la résine alkyde "BECKOSOL ODE 230-70-E" de la société DAINIPPON, la résine acrylique "ACRYLOID B66" de la société ROHM & HAAS, la résine polyuréthane "TRIXENE PR 4127" de la société BAXENDEN.

Le polymère filmogène additionnel peut être un des polymères décrits dans la demande FR0450540 déposée le 18 mars 2004, dont le contenu est inclus dans la présente demande par référence.

Le polymère filmogène additionnel présent dans la composition selon l'invention en une teneur allant de 0,1 % à 60 % en poids, par rapport au poids total de la composition, de préférence allant de 2 % à 40 % en poids, et mieux de 5 % à 25 % en poids.

La première composition peut comprendre, en outre, au moins un agent plastifiant tel que décrit dans la demande FR0314654 déposée le 12 décembre 2003, dont le contenu est inclus dans la présente demande par référence. La quantité de plastifiant peut être choisie par l'homme du métier sur la base de ses connaissances générales, de manière à obtenir une composition ayant des propriétés cosmétiquement acceptables.

## Seconde composition

Le produit cosmétique selon l'invention comprend une seconde composition contenant un milieu cosmétiquement acceptable.

La seconde composition est avantageusement choisie de telle manière qu'elle améliore au moins une propriété cosmétique de la première composition, lorsque celle-ci est appliquée seule sur ladite matière kératinique. La deuxième composition peut notamment améliorer le confort de la première composition ou diminuer son caractère collant.

La seconde composition peut également être choisie de manière à ce que le produit, une fois appliqué sur les matières kératiniques, présente des propriétés de brillance satisfaisantes.

La première composition et/ou la seconde composition, peuvent être choisies de manière à ce que le produit, une fois appliqué sur les matières kératiniques, présente des propriétés de brillance satisfaisantes.

*Brillance*

La brillance moyenne du produit mesurée à 20°, une fois la première et la deuxième compositions étalées l'une sur l'autre sur un support, peut être avantageusement supérieure ou égale à 30, de préférence supérieure ou égale à 40, de préférence supérieure ou égale à 50, de préférence supérieure ou égale à 60 sur 100.

Par « brillance moyenne », on désigne la brillance telle qu'elle peut être mesurée à l'aide d'un brillancemètre, de manière conventionnelle par la méthode suivante.

On peut mesurer la brillance d'un dépôt de la première composition seule, la brillance d'un dépôt de la deuxième composition seule, ou la brillance d'un dépôt de produit selon l'invention constitué de la superposition de dépôts de la première et de la deuxième compositions.

Sur une carte de contraste de marque LENETA et de référence FORM 1A PENOPAC, on étale une couche comprise entre 50 µm et 150 µm d'épaisseur d'une composition à l'aide d'un étaleur automatique.

La couche de composition ou de produit recouvre au moins le fond blanc de la carte. On laisse sécher le dépôt 24 heures à une température de 30°C, puis on procède à la mesure de la brillance à 20° sur le fond blanc à l'aide d'un brillancemètre de marque BYK GARDNER et de référence microTRI-GLOSS.

Cette mesure (comprise entre 0 et 100) est répétée au moins trois fois, et la brillance moyenne est la moyenne des au moins trois mesures effectuées.

Lorsqu'on souhaite mesurer la brillance du produit selon l'invention, on étale une première couche d'une des deux compositions sur la carte de contraste, dans les mêmes conditions que précédemment décrites. On laisse sécher cette première couche le temps nécessaire, puis on étale par dessus une deuxième couche de l'autre composition, dans les mêmes conditions que précédemment décrites.

La brillance moyenne de la première composition, ou de la deuxième composition, ou du produit de l'invention, mesurée à 20° est avantageusement supérieure ou égale à 30, mieux supérieure ou égale à 35, mieux encore supérieure ou égale à 40, mieux encore supérieure

ou égale à 45, mieux encore supérieure ou égale à 50 sur 100, mieux encore, supérieure ou égale à 55, mieux encore, supérieure ou égale à 60, mieux encore, supérieure ou égale à 65, mieux encore, supérieure ou égale à 70 ou mieux encore, supérieure ou égale à 75 sur 100. Pour certaines compositions selon l'invention, telles que des vernis à ongles, la brillance mesurée à 20° peut être supérieure ou égale à 70, voire 80 sur 100.

De préférence, la brillance moyenne de la première composition, ou de la deuxième composition, ou du produit de l'invention, une fois étalée sur un support, mesurée à 60° est supérieure ou égale à 50, mieux encore, supérieure ou égale à 60, mieux encore, supérieure ou égale à 65, mieux encore, supérieure ou égale à 70, mieux encore, supérieure ou égale à 75, mieux encore, supérieure ou égale à 80, mieux encore, supérieure ou égale à 85 ou mieux encore, supérieure ou égale à 90 sur 100.

On procède à la mesure de la brillance moyenne à 60° dans les mêmes conditions que celles décrites précédemment pour mesurer la brillance moyenne à 20°.

*Non transfert*

La première composition et/ou la seconde composition, peuvent également être choisies de manière à ce que le produit, une fois appliqué sur les matières kératiniques, présente des propriétés de non transfert satisfaisantes.

En particulier, la première composition a de préférence un indice de transfert inférieur à 35 de préférence inférieur ou égal à 30, de préférence inférieur ou égal à 20, de préférence inférieur ou égal à 15, de préférence inférieur ou égal à 10, de préférence, inférieur ou égal à 5, et de préférence encore inférieur ou égal à 2 sur 100.

La seconde composition peut être choisie de telle sorte que l'indice de transfert du produit de l'invention est inférieur ou égal à 35, de préférence inférieur ou égal à 30, de préférence inférieur ou égal à 20, de préférence inférieur ou égal à 15, de préférence inférieur ou égal à 10, de préférence, inférieur ou égal à 5.

L'indice de transfert peut être mesuré selon la méthode suivante.

On préchauffe un support (rectangle de 40 mm X 70 mm et d'épaisseur 3 mm) de mousse de polyéthylène adhésif sur une des faces ayant une densité de 33 kg/m3 (vendue sous la dénomination RE40X70EP3 de la société JOINT TECHNIQUE LYONNAIS IND) sur une plaque chauffante maintenue à la température de 40 °C pour que la surface du support soit maintenue à une température de 33 °C ± 1 °C.

Tout en laissant le support sur la plaque chauffante, on applique la composition sur toute la surface non adhésive du support en l'étalant à l'aide d'un pinceau pour obtenir un dépôt de la composition d'environ 15 µm puis on laisse sécher pendant 30 minutes.

Après séchage, le support est collé par sa face adhésive sur une enclume d'un diamètre de 20 mm et munie d'un pas de vis. L'ensemble support/dépôt est ensuite découpé à l'aide d'un emporte- pièce d'un diamètre de 18 mm. L'enclume est ensuite vissée sur une presse (STATIF MANUEL IMADA SV-2 de la société SOMECO) équipée d'un dynanomètre (IMADA DPS-20 de la société SOMECO).

Un papier blanc pour photocopieuse de 80 g/m2 est placé sur le socle de la presse puis on presse l'ensemble support/dépôt sur le papier à une pression de 2,5 kg pendant 30 secondes. Après retrait de l'ensemble support/dépôt, une partie du dépôt a transféré sur le papier. On mesure alors la couleur du dépôt transféré sur le papier à l'aide d'un colorimètre MINOLTA CR300, la couleur étant caractérisé par les paramètres colorimétriques L*, a*, b*. On détermine les paramètres colorimétriques $L^*_0$, $a^*_0$, $b^*_0$ de la couleur du papier nu utilisé.

On détermine alors la différence de couleur ΔE1 entre la couleur du dépôt transféré par rapport à la couleur du papier nu par la relation suivante.

$$\Delta E1 = \sqrt{(L^*-L_o^*)^2 + (a^* - a_o^*)^2 + (b^* - b_o^*)^2}$$

Par ailleurs, on prépare une référence de transfert total en appliquant la composition directement sur un papier identique à celui utilisé précédemment, à la température ambiante (25 °C), en étalant la composition à l'aide d'un pinceau et pour obtenir un dépôt de la composition d'environ 15 µm puis on laisse sécher pendant 30 minutes à la température ambiante (25 °C). Après séchage, on mesure directement les paramètres colorimétriques L*', a*', b*' de la couleur du dépôt mis sur le papier, correspondant à la couleur de référence de transfert total. On détermine les paramètres colorimétriques $L^{*'}_0$, $a^{*'}_0$, $b^{*'}_0$ de la couleur du papier nu utilisé.

On détermine alors la différence de couleur ΔE2 entre la couleur de référence de transfert total par rapport à la couleur du papier nu par la relation suivante.

$$\Delta E2 = \sqrt{(L^{*'}-L_o^{*'})^2 + (a^{*'} - a_o^{*'})^2 + (b^{*'} - b_o^{*'})^2}$$

Le transfert de la composition, exprimé en pourcentage, est égal au rapport :

$$100 \times \Delta E1 / \Delta E2$$

La mesure est effectuée sur 4 supports à la suite et la valeur de transfert correspond à la moyenne des 4 mesures obtenues avec les 4 supports.

*Tenue*

La première composition et/ou la seconde composition, peut également être choisie de manière à ce que le produit, une fois appliqué sur les matières kératiniques, présente des propriétés de tenue satisfaisantes.

En particulier, la première composition a de préférence ayant un indice de tenue supérieur ou égal à 80 %, de préférence supérieur ou égal à 85 %, de préférence inférieur ou égal à 90 %, de préférence supérieur ou égal à 95 %.

La seconde composition peut être choisie de telle sorte que l'indice de tenue du produit est supérieur ou égal à 80 %, de préférence supérieur ou égal à 85 %, de préférence inférieur ou égal à 90 %, de préférence supérieur ou égal à 95 %.

L'indice de tenue du dépôt obtenue avec la composition selon l'invention est déterminé selon le protocole de mesure décrit ci-après.

On prépare un support (rectangle de 40 mm X 70 mm) constitué d'un revêtement acrylique (adhésif acrylique hypoallergénique sur film polyéthylène vendu sous la dénomination BLENDERME ref FH5000-55113 par la société 3M Santé) collé sur une couche de mousse de polyéthylène adhésif sur la face opposée à celle sur laquelle est fixé le sparadrap (couche de mousse vendue sous la dénomination RE40X70EP3 de la société JOINT TECHNIQUE LYONNAIS IND).

On mesure à l'aide d'un colorimètre MINOLTA CR 300 la couleur $L^*_0 a^*_0 b^*_0$ du support , côté face revêtement acrylique.

On préchauffe le support ainsi préparé sur une plaque chauffante maintenue à la température de 40 °C pour que la surface du support soit maintenue à une température de 33 °C ± 1 °C.

Tout en laissant le support sur la plaque chauffante, on applique la composition sur toute la surface non adhésive du support (c'est-à-dire sur la surface du revêtement acrylique) en l'étalant à l'aide d'un pinceau pour obtenir un dépôt de la composition d'environ 15 µm puis on laisse sécher pendant 10 minutes.

Après séchage, on mesure la couleur L*a*b* du film ainsi obtenu.

On détermine alors la différence de couleur ΔE1 entre la couleur du film par rapport à la couleur du support nu par la relation suivante.

$$\Delta E1 = \sqrt{(L^*-L_o^*)^2 + (a^* - a_o^*)^2 + (b^* - b_o^*)^2}$$

Le support est ensuite collé par sa face adhésive (face adhésive de la couche de mousse) sur une enclume d'un diamètre de 20 mm et munie d'un pas de vis. Une éprouvette de l'ensemble support/dépôt est ensuite découpée à l'aide d'un emporte- pièce d'un diamètre de 18 mm. L'enclume est ensuite vissée sur une presse (STATIF MANUEL IMADA SV-2 de la société SOMECO) équipée d'un dynanomètre (IMADA DPS-20 de la société SOMECO).

Sur un papier blanc pour photocopieuse de grammage 80g/m2 , on dessine une bande de 33 mm de largeur et 29,7 cm de longueur, on trace un premier trait à 2 cm du bord de la feuille, puis un deuxième trait à 5 cm du bord de la feuille, les premier et deuxième traits délimitant ainsi une case sur la bande ; puis on dispose une première marque et une deuxième marque situées dans la bande respectivement aux repères 8 cm et 16 cm du deuxième trait . On place sur la première marque 20 µl d'eau et sur la deuxième marque 10 µl d'huile de tournesol raffinée (vendue par la société LESIEUR).

Le papier blanc est placé sur le socle de la presse puis on presse l'éprouvette placée sur la case de la bande de papier à une pression d'environ 300 g/cm$^2$ exercée pendant 30 secondes. Puis on relève la presse et on place à nouveau l'éprouvette juste après le deuxième trait (donc à côté de la case), on effectue à nouveau une pression d'environ 300 g/cm$^2$ et on déplace, de manière rectiligne dès le contact effectué, le papier avec une vitesse de 1 cm/ s, sur toute la longueur de la bande de telle sorte que l'éprouvette traverse les dépôts d'eau et d'huile.

Après retrait de l'éprouvette, une partie du dépôt a transféré sur le papier. On mesure alors la couleur L*', a*', b*' du dépôt resté sur l'éprouvette.

On détermine alors la différence de couleur ΔE2 entre la couleur du dépôt resté sur l'éprouvette par rapport à la couleur du support nu par la relation suivante.

$$\Delta E2 = \sqrt{(L^{*\prime}-L_o^*)^2 + (a^{*\prime} - a_o^*)^2 + (b^{*\prime} - b_o^*)^2}$$

L'indice de tenue de la composition, exprimée en pourcentage, est égale au rapport :

$$100 \times \Delta E2/\Delta E1$$

La mesure est effectuée sur 6 supports à la suite et la valeur de transfert correspond à la moyenne des 6 mesures obtenues avec les 6 supports.

Le milieu cosmétiquement acceptable de la seconde composition comprend de préférence une phase liquide non volatile à température ambiante et pression atmosphérique.

Par « phase liquide non volatile », on entend tout milieu susceptible de rester sur la peau ou les lèvres pendant plusieurs heures. Une phase liquide non volatile a en particulier une pression de vapeur à température ambiante et pression atmosphérique, non nulle inférieure à 0,02 mm de Hg (2,66 Pa) et mieux inférieure à $10^{-3}$ mm de Hg (0,13 Pa).

La phase liquide non volatile de la seconde composition peut être au moins une phase hydrocarbonée, une phase siliconée liquide, une phase fluorée liquide à température ambiante, ou un de leurs mélanges.

Par "phase liquide non volatile hydrocarbonée", on entend une phase comprenant au moins une huile contenant majoritairement des atomes de carbone et des atomes d'hydrogène et en particulier des chaînes alkyle ou alcényle comme les alcanes ou alcènes mais aussi les huiles à chaîne alkyle ou alcényle comportant un ou des groupements éther, ester ou acide carboxylique.

La phase liquide non volatile de la seconde composition représente avantageusement 1 à 100 %, de préférence de 5 à 95 %, mieux de 20 à 80 % et mieux encore, de 40 à 80 % du poids total de la seconde composition.

*Huile siliconée non volatile de la phase liquide*

La phase liquide non volatile de la seconde composition contient avantageusement au moins une huile siliconée, par exemple une huile siliconée phénylée ou une huile polydiméthylsiloxane.

L'huile siliconée non volatile peut être choisie parmi les polyalkylsiloxanes, les polyarylsiloxanes, les polyalkylarylsiloxanes et leurs mélanges.

L'huile siliconée non volatile peut être choisie parmi

- les polydiméthylsiloxanes (PDMS) non volatils, linéaires ou ramifiés ;
- les polydiméthylsiloxanes comportant des groupements alkyle, alcoxy ou phényle, pendant ou en bout de chaîne siliconée, groupements ayant de 2 à 24 atomes de carbone ;

- les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.

Les polyalkylsiloxanes selon l'invention incluent les polydiméthylsiloxanes, les copolymères (polydiméthylsiloxane)(méthylvinylsiloxane), les poly(diméthylsiloxane)(diphényl)siloxanes, les copolymères poly(diméthylsiloxane)(diphényl)(méthylvinylsiloxane), et leurs mélanges

On préfère comme huile siliconée non volatile, une huile choisie parmi les silicones de formule (II):

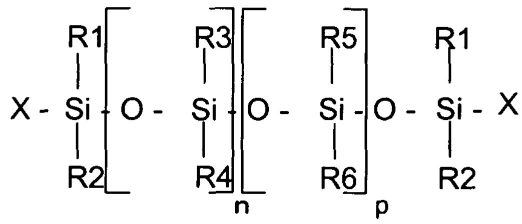

(II)

dans laquelle :

$R_1$, $R_2$, $R_5$ et $R_6$ sont, ensemble ou séparément, un radical alkyle ayant 1 à 6 atomes de carbone,
$R_3$ et $R_4$ sont, ensemble ou séparément, un radical alkyle ayant de 1 à 6 atomes de carbone, ou un radical aryle,
X est un radical alkyle ayant de 1 à 6 atomes de carbone, un radical hydroxyle ou un radical vinyle,
n et p étant choisis de manière à conférer à l'huile une masse moléculaire en poids inférieure à 200 000 g/mol, de préférence inférieure à 150 000 g/mol et de préférence encore inférieure à 100 000 g/mol.

On choisit par exemple comme huile siliconée non volatile de formule (II) une polydiméthylsiloxane de viscosité comprise entre 0,5 et 60 000 cSt, de préférence entre 0,5 et 10 000 cSt, de préférence entre 0,5 et 1 000 cSt mesurée selon la norme ASTM D-445, par exemple la DC 200 de viscosité 350 cSt, commercialisée par Dow Corning.

Selon un mode de mise en oeuvre, la deuxième composition contient une huile siliconée non volatile de formule (II) telle qu'une polydiméthylsiloxane de viscosité comprise de préférence entre 0,5 et 500 cSt, de préférence encore entre 1 et 10 cSt, par exemple la polydiméthylsiloxane vendue sous la dénomination DC 200 de viscosité 5 cSt et de poids moléculaire 800, commercialisée par Dow Corning.

La masse moléculaire en poids de l'huile siliconée non volatile est de préférence comprise entre 400 et 200 000, de préférence entre 4 000 et 100 000, de préférence entre 4 000 et 20 000, de préférence compris entre 400 et 2 000, de préférence encore entre 400 et 1 000 g/mol.

*Huile fluorée non volatile de la phase liquide*

La phase liquide non volatile de la seconde composition peut comprendre au moins une huile fluorée de formule (III) :

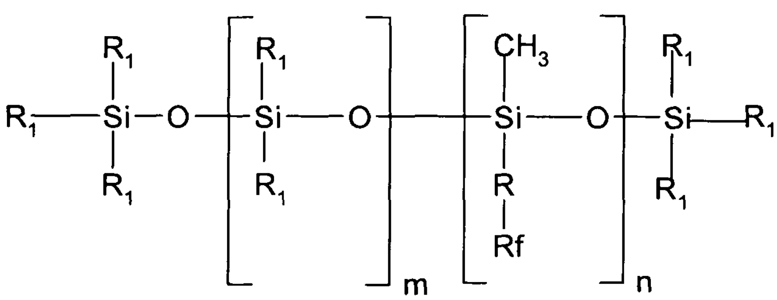

(III)

dans laquelle :

- R représente un groupement alkylényle linéaire ou ramifié, ayant 1 à 6 atomes de carbone, de préférence un groupement divalent méthylényle, éthylényle, propylényle ou butylényle,
- Rf représente un radical fluoroalkyle, notamment un radical perfluoroalkyle,

ayant 1 à 9 atomes de carbone, de préférence 1 à 4 atomes de carbone,

- $R_1$ représentent, indépendamment l'un de l'autre, un radical alkyle en $C_1$-$C_{20}$, de préférence en $C_1$-$C_4$, un radical hydroxyle, ou un radical phényle,
- m est choisi de 0 à 150, de préférence de 20 à 100, et
- n est choisi de 1 à 300, de préférence de 1 à 100.

On préfère les huiles de formule (III) telles que R1 est un méthyle, R est un éthyle et Rf est CF3. On peut notamment citer comme composés fluorosiliconés d e formule (III) ceux qui sont commercialisés par la société Shin Etsu sous les dénominations 'X22-819', 'X22-820', 'X22-821' et 'X22-822' ou encore 'FL-100'.
On peut également citer parmi les huiles fluorées, les polyéthers fluorés choisis parmi les composé de formule (IV) suivante :

$$R_6 - (CF_2\text{-}CFR_3\text{-}CF_2O)p - (CFR_4\text{-}CF_2\text{-}O)q - (CFR_5\text{-}O)r - R_7 \qquad \text{(IV)}$$

dans laquelle :

- $R_3$ à $R_6$ représentent, de manière indépendante l'un de l'autre, un radical monovalent choisi parmi -F, -$(CF_2)$n-$CF_3$, et -O-$(CF_2)$n-$CF_3$,
- $R_7$ représente un radical monovalent choisi parmi -F et -$(CF_2)$n-$CF_3$,
- avec n allant de 0 à 4 inclus,
- p allant de 0 à 600, q allant de 0 à 860, r allant de 0 à 1500, et p, q et r étant des entiers choisis de manière à ce que la masse moléculaire en poids du composé va de 500 à 100000, de préférence de 500 à 10000.

Les huiles fluorées peuvent également être choisies parmi les alcanes fluorés sont choisi parmi les perfluoroalcanes et les fluoroalcanes en C2-C50, de préférence en C5-C30, tels que la perfluorodécaline, le perfluoroadamantane et le bromoperfluorooctyle et leurs mélanges.

*Huile volatile de la phase liquide*

La deuxième composition peut éventuellement contenir une huile volatile.
Ces huiles peuvent être des huiles hydrocarbonées, des huiles siliconés comportant éventuellement des groupements alkyle ou alkoxy pendants ou en bout de chaîne siliconée ou un mélange de ces huiles.
De préférence, les huiles volatiles sont des huiles cosmétiques choisies parmi les huiles ayant un point éclair aliant de 40°C à 100°C, et leurs mélanges. En outre, ils présentent avantageusement une température d'ébullition à pression atmosphérique inférieure à 220°C et mieux inférieure à 210°C, notamment allant de 110 à 210°C. De préférence, ces huiles volatiles ne sont pas des monoalcools à au moins 7 atomes de carbone.
Comme huile volatile utilisable dans l'invention, on peut citer les huiles de silicones linéaires ou cycliques ayant une viscosité à température ambiante inférieure à 8 cSt et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthylcyclo-tétrasiloxane, le décaméthylcyclopentasiloxane, le dodécaméthylcyclohexasiloxane, l'heptaméthyl hexyltrisiloxane, l'heptaméthyloctyl trisiloxane, l'hexaméthyldisiloxane, l'octaméthyl trisiloxane, le décaméthyl tétrasiloxane, le dodécaméthyl pentasiloxane et leurs mélanges.
Comme autre huile volatile utilisable dans l'invention, on peut citer les huiles volatiles hydrocarbonées ayant de 8 à 16 atomes de carbone et leurs mélanges et notamment les alcanes ramifiés en $C_8$-$C_{16}$ comme les isoalcanes (appelés aussi isoparaffines) en $C_8$-$C_{16}$, l'isododécane, l'isodécane, l'isohexadécane et par exemple les huiles vendues sous les noms commerciaux d'Isopars' ou de Permetyls, les esters ramifiés en $C_8$-$C_{16}$ comme le néo pentanoate d'isohexyle et leurs mélanges.
De préférence, on utilise l'isododécane (Permetyls 99 A), les isoparaffines en $C_8$-$C_{16}$ comme l'Isopar L, E, G ou H, leurs mélanges, éventuellement associés au décaméthyl tétrasiloxane ou au cyclopentasiloxane.
De façon générale, la quantité d'huile volatile est utilisée en une quantité suffisante pour améliorer les qualités

d'étalement de la deuxième composition. Cette quantité sera adaptée par l'homme du métier en fonction de l'intensité des propriétés recherchées.

La quantité d'huile volatile est choisie de manière à ne pas diminuer la brillance de la deuxième composition. Selon un mode de réalisation, la deuxième composition ne contient pas d'huile volatile.

*Polymère de haut poids moléculaire*

La deuxième composition contient avantageusement un polymère de haut poids moléculaire, différent de l'huile siliconée non volatile décrite précédemment.

Lorsque la deuxième composition selon l'invention est liquide, elle comprend avantageusement 20 à 50 % en poids d'un polymère de haut poids moléculaire.

Selon un mode de mise en oeuvre, la deuxième composition contient avantageusement le mélange d'une polydiméthylsiloxane de masse moléculaire comprise entre 200 000 et 300 000 g/mol et d'une polydiméthylsiloxane de masse moléculaire comprise entre 400 et 1 000 g/mol.

La proportion massique du composé siliconé de haut poids moléculaire/composé siliconé liquide est de préférence comprise entre 20/80 et 60/40, de préférence entre 35/65 et 45/55.

Lorsque la deuxième composition selon l'invention est solide, elle comprend avantageusement de 2 à 40 % en poids d'un polymère de haut poids moléculaire.

Le polymère est de préférence un polymère siliconé.

Ce polymère peut être liquide ou solide à température ambiante et sa masse moléculaire en poids est supérieure ou égale à 200 000 g/mol, de préférence compris entre 200 000 et 2 500 000, de préférence entre 200 000 et 2 000 000 g/mol. Lorsque le polymère est liquide, il fait partie de la phase liquide non volatile de la deuxième composition.

La viscosité de ce polymère peut être comprise entre 10 000 et 5 000 000 cSt, de préférence entre 100 000 et 1 000 000 cSt, de préférence encore entre 300 000 et 700 000 cSt, mesurée selon la norme ASTM D-445.

Le polymère de haut poids moléculaire est avantageusement un polymère non greffé, c'est à dire un polymère obtenu par polymérisation d'au moins un monomère, sans réaction subséquente des chaînes latérales avec un autre composé chimique. Le polymère est de préférence choisi parmi les diméthiconols, les fluorosilicones, les diméthicones et leurs mélanges. Le polymère est de préférence un homopolymère.

En particulier, on peut utiliser un polymère de haut poids moléculaire répondant à la formule (V):

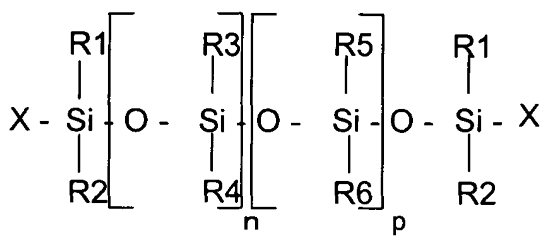

(V)

dans laquelle :

$R_1$, $R_2$, $R_5$ et $R_6$ sont, ensemble ou séparément, un radical alkyle ayant 1 à 6 atomes de carbone, éventuellement substitué par au moins un atome de fluor,

$R_3$ et $R_4$ sont, ensemble ou séparément, un radical alkyle ayant de 1 à 6 atomes de carbone ou un radical aryle,

X est un radical alkyle ayant de 1 à 6 atomes de carbone, un radical hydroxyle, un radical vinyle, allyle ou un radical alkoxyle ayant de 1 à 6 atomes de carbone,

n et p étant choisis de manière à ce que le composé siliconé ait une masse moléculaire en poids supérieure ou égale à 200 000 g/mol.

p est de préférence égal à 0.

Les polymères de formule (V) tels $R_1$ à $R_6$ représentent un groupement méthyle et le substituant X représente un groupement hydroxyle sont des diméthiconols. On cite par exemple les polymères de formule (V) tels que p=0 et n est compris entre 2 000 et 40 000, de préférence entre 3 000 et 30 000. On peut également citer les polymères ayant une masse moléculaire comprise entre 1 500 000 et 2 000 000 g/mol.

Selon un mode de mise en oeuvre, le polymère de haut poids moléculaire est la diméthiconol commercialisée

par Dow Corning dans une polydiméthylsiloxane (5 cSt) sous la référence D2-9085, la viscosité du mélange étant égale à 1 550 cSt, ou la diméthiconol commercialisée par Dow Corning dans une polydiméthylsiloxane (5cS) sous la référence DC 1503. On préfère également la diméthiconol (de poids moléculaire égal à 1 770 000 g/mol) commercialisée par Dow Corning sous la référence Q2-1403 ou Q2-1401, la viscosité du mélange étant égale à 4 000 cSt.

Comme polymère de haut poids moléculaire utilisable selon l'invention, on peut citer ceux pour lesquels :

- les substituants $R_1$ à $R_6$ et X représentent un groupement méthyle, comme celle vendue sous la dénomination SE30 par la société Général Electric, et celle vendue sous la dénomination AK 500000 par la société Waker,
- les substituants $R_1$ à $R_6$ et X représentent un groupement méthyle, p et n sont tels que le poids moléculaire est de 250 000 g/mol, comme celle vendue sous la dénomination Silbione 70047 V par la société Rhodia,
- les substituants $R_1$ à $R_6$ représentent un groupement méthyle, le substituant X représente un groupement hydroxyle, comme celle vendue sous la dénomination Q2-1401 ou Q2-1403 par la société Dow Corning,
- les substituants $R_1$, $R_2$, $R_5$, $R_6$ et X représentent un groupement méthyle, les substituants $R_3$ et $R_4$ représentent un groupement aryle, n et p sont tels que le poids moléculaire du polymère soit de 600 000 g/mol, comme celle vendue sous la dénomination 761 par la société Rhône-Poulenc.

Le polymère siliconé de haut poids moléculaire est de préférence introduit dans la composition sous la forme d'un mélange avec une silicone liquide, la viscosité de ladite silicone liquide étant comprise entre 0,5 et 10 000 cSt, de préférence entre 0,5 et 500 cSt, de préférence encore entre 1 et 10 cSt.

La silicone fluide peut être choisie parmi les polyalkylsiloxanes, les polyarylsiloxanes, les polyalkylarylsiloxanes et leurs mélanges. La silicone liquide peut être une silicone volatile telle qu'une polydiméthylsiloxane cyclique comprenant 3 à 7 motifs $-(CH_3)_2SiO-$.

La silicone liquide peut également être une silicone non volatile polydiméthylsiloxane, notamment de viscosité comprise entre 0,5 et 10 000 cSt, de préférence encore de l'ordre de 5 cSt, par exemple la silicone commercialisée sous la référence DC 200 par Dow Corning.

La proportion du polymère siliconé de haut poids moléculaire dans le mélange polymère siliconé de haut poids moléculaire/silicone liquide est de préférence compris entre 10/90 et 20/80. La viscosité du mélange polymère siliconé de haut poids moléculaire/silicone liquide est de préférence comprise entre 1 000 et 10 000 cSt.

Les diméthicones de haut poids moléculaire selon l'invention incluent les diméthicones décrites d ans le brevet US 4 1 52 416. Elles sont par exemple commercialisées sous les références SE30, SE33, SE 54 et SE 76.

Les diméthicones selon l'invention sont par exemple des composés de formule (III) telle que $R_1$ à $R_6$ et X sont des méthyles et p=0. Le poids moléculaire de ces polymères est de préférence compris entre 200 000 et 300 000, de préférence entre 240 000 et 260 000 g/mol.

Les diméthicones selon l'invention incluent les polydiméthylsiloxanes, les copolymères (polydiméthylsiloxane) (méthylvinylsiloxane), les copolymères poly(diméthylsiloxane) (diphényl)(méthylvinylsiloxane), et leurs mélanges.

Les fluorosilicones de haut poids moléculaire selon l'invention ont de préférence un poids moléculaire compris entre 200 000 et 300 000, de préférence entre 240 000 et 260 000 g/mol.

Selon un des aspects de l'invention, la deuxième composition selon l'invention comprend au moins un composé apolaire ou peu polaire qui peut être choisi parmi les huiles, les gommes et/ou les cires. La deuxième composition comprend de préférence plus de 70 %, de préférence plus de 80 % en poids, de préférence 100 % en poids de composés apolaires ou peu polaires. Ces composés apolaires ou peu polaires comprennent des agents de coloration ou des agents gélifiants.

Selon un mode de réalisation, la seconde composition comprend le mélange d'un polymère ayant une masse moléculaire en poids supérieure ou égale à 200 000 g/mol et une huile siliconée telle que décrite précédemment.

Selon un mode de réalisation, la deuxième composition est transparente.

On entend par « composition transparente », une composition transparente à translucide, c'est-à-dire telle qu'elle transmet au moins 40 % de lumière à une longueur d'onde de 750 nm, et de préférence au moins 50 % de lumière.

La mesure de la transmission est effectuée avec un spectrophotomètre UV-visible Cary 300 Scan de la société Varian selon le protocole suivant :

On coule la composition au-dessus de sa température de fusion dans une cuve à spectrophotomètre à section carrée dont le côté a une longueur de 10 mm.

L'échantillon de la composition est ensuite refroidi pendant 24 heure à 35 °C puis maintenu dans une enceinte thermostatée à 20 °C pendant 24 heures.

On mesure ensuite la lumière transmise à travers l'échantillon de la composition au spectrophotomètre par balayage de longueurs d'onde allant de 700 nm à 800 nm, la mesure étant faite en mode transmission.

On détermine alors le pourcentage de lumière transmise à travers l'échantillon de la composition à la longueur d'onde de 750 nm.

Lorsque la seconde composition est transparente, elle contient avantageusement moins de 5% de pigments, de préférence moins de 2%, de préférence encore moins de 1%.

Le mélange polymère de haut poids moléculaire/composé siliconé liquide représente de préférence plus de 70%, de préférence plus de 80% en poids, de préférence plus de 90% en poids, de préférence encore plus de 95% en poids de la deuxième composition.

La deuxième composition peut comprendre d'autres composés, qui sont de préférence apolaires ou peu polaires. Ces composés apolaires ou peu polaires sont de préférence des composés siliconés, des agents de coloration ou des agents gélifiants.

Selon un mode de mise en oeuvre, la deuxième composition ne comprend que des ingrédients apolaires ou peu polaires.

La deuxième composition peut comprendre au moins une cire, notamment lorsqu'elle est sous forme solide.

Les cires peuvent être présentes à raison de 0,5-30% en poids dans la composition et mieux de 5 à 20%, de préférence entre 5 et 15%, de la composition.

On préfère dans le cadre de la présente invention des cires hydrocarbonées linéaires. Leur point de fusion est avantageusement supérieur à 35°C, par exemple supérieur à 55°C, de préférence supérieur à 80°C.
Les cires hydrocarbonées linéaires sont avantageusement choisies parmi les alcanes linéaires substitués, les alcanes linéaires non substitués, les alcènes linéaires non substitués, les alcènes linéaires substitués, un composé non substitué étant uniquement composé de carbone et d'hydrogène. Les substituants mentionnés précédemment ne contenant pas d'atomes de carbone.
Les cires hydrocarbonées linéaires incluent les polymères et copolymères de l'éthylène, de poids moléculaire compris entre 400 et 800, par exemple la Polywax 500 ou Polywax 400 commercialisée par New Phase Technologies.
Les cires hydrocarbonées linéaires incluent les cires de paraffine linéaires, comme les paraffines S&P 206, S&P 173 et S&P 434 de Strahl & Pitsch.

Les cires hydrocarbonées linéaires incluent les alcools linéaires à longue chaîne, comme les produits comprenant un mélange de polyéthylène et d'alcools en comprenant 20 à 50 atomes de carbones, notamment le Performacol 425 ou le Performacol 550 (mélange dans les proportions 20/80) commercialisés par New Phase Technologies.

La deuxième composition contient avantageusement une cire siliconée, telle qu'une diméthicone comprenant des groupements alkyles en bout de chaîne. Ces groupements alkyles ont de préférence plus de 18 atomes de carbones, de préférence entre 20 et 50, de préférence entre 30 et 45 atomes de carbone.

La cire siliconée répond par exemple à la formule (VI) ou (VII)

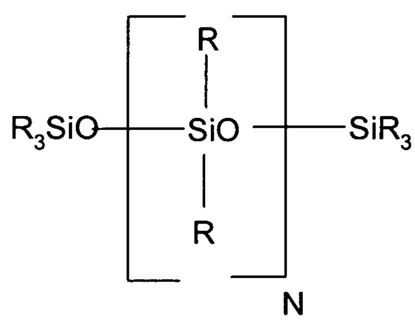

(VI)

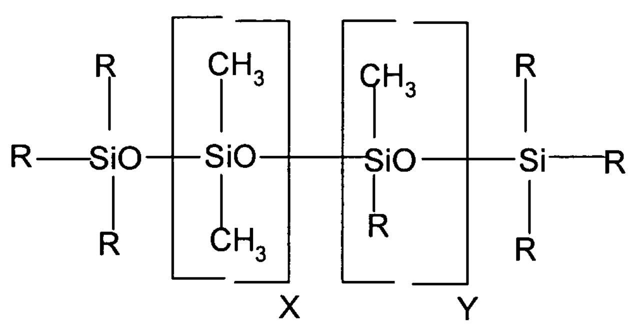

(VII)

dans lesquelles R est un alkyle, X est égal ou supérieur à zéro, et N et Y sont égaux ou supérieurs à un.

R comprend de 1 à 50 atomes de carbone à la condition que le compose soit solide à température ambiante.

Des exemples de cires siliconées sont par exemple

- Les C20-24 alkyl méthicone, C24-28 alkyl diméthicone, C20-24 alkyl diméthicone, C24-28 alkyl diméthicone commercialisées par Archimica Fine Chemicals sous la référence SilCare 41 M40, SilCare 41 M50, SilCare 41 M70 and SilCare 41 M80,
- Les stéaryl diméthicone de référence SilCare 41 M65 commercialisées par Archimica ou de référence DC-2503 commercialisée par Dow-Corning
- Les stéaroxytriméthylsilane vendues sous la référence SilCare 1 M71 ou DC-580
- Les produits ABIL Wax 9810, 9800, or 2440 de Wacker-Chemie GmbH,
- Les C30-45 alkyl méthicone commercialisées par Dow Corning sous la référence AMS-C30 Wax, de même que les C30-45 alkyl diméthicone commercialisées par General Electric sous la référence SF1642 ou SF-1632.

Lorsque la première ou la deuxième composition est destinée à être appliquée sur les ongles, la composition contient de préférence un solvant choisi parmi:

- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la γ-butyrolactone ;
- les esters à chaîne courte (ayant de 3 à 8 atomes de carbone au total) tels que l'acétate d'éthyle, l'acétate de méthyle, l'acétate de propyle, l'acétate d'isopropyl, l'acétate de n-butyle, l'acétate d'isopentyle, l'acétate de méthoxypropyle, le lactate de butyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde ;
- les composés hétérocycliques tels que le tétrahydrofuranne ;
- le carbonate de propylène, le 3-éthoxypropionate d'éthyle ;
- leurs mélanges.

On préfère en particulier l'acétate de méthyle, l'acétate d'isopropyle, l'acétate de méthoxypropyle, le lactate de butyle, l'acétone, la méthyléthylcétone, le diacétone alcool, la γ-butyrolactone, le tétrahydrofuranne, le carbonate de propylène, le 3-éthoxypropionate d'éthyle, le diméthylsulfoxide, et leurs mélanges.

La première et/ou la deuxième composition du produit cosmétique selon l'invention peut contenir un agent de coloration qui peut être choisi parmi les colorants hydrosolubles ou liposolubles, les pigments, les nacres et leurs mélanges.

Par « pigments », il faut comprendre des particules blanches ou colorées, minérales ou organiques, insolubles dans la phase organique liquide, destinées à colorer et/ou opacifier la première composition.

Par « nacres », il faut comprendre des particules irisées, notamment produites par certains mollusques dans leur coquille ou bien synthétisées, insolubles dans le milieu de la première composition.

Par « colorants », il faut comprendre des composés généralement organiques solubles dans les corps gras comme les huiles ou dans une phase hydroalcoolique.

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Les pigments, les nacres ou les charges solides peuvent être dispersées dans la phase grasse liquide de la composition en présence d'un agent dispersant.

L'agent dispersant sert à protéger les particules dispersées contre leur agglomération ou floculation. Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent

s'accrocher physiquement ou chimiquement à la surface des pigments. Ces dispersants présentent, en outre, au moins un groupe fonctionnel compatible ou soluble dans le milieu continu. En particulier, on utilise les esters de l'acide hydroxy-12 stéarique en particulier et d'acide gras en $C_8$ à $C_{20}$ et de polyol comme le glycérol, la diglycérine, tel que le stéarate d'acide poly(12-hydroxystéarique) de poids moléculaire d'environ 750g/mole tel que celui vendu sous le nom de Solsperse 21 000 par la société Avecia, le polygycéryl-2 dipolyhydroxystéarate (nom CTFA) vendu sous la référence Dehymyls PGPH par la société Henkel ou encore l'acide polyhydroxystéarique tel que celui vendu sous la référence Arlacel P100 par la société Uniqema et leurs mélanges.

Comme autre dispersant utilisable dans la composition de l'invention, on peut citer les dérivés ammonium quaternaire d'acides gras polycondensés comme le Solsperse 17 000 vendu par la société Avecia, les mélanges de poly diméthylsiloxane/oxypropylène tels que ceux vendus par la société Dow Corning sous les références DC2-5185, DC2-5225 C.

L'acide polydihydroxystéarique et les esters de l'acide hydroxy12-stéarique sont de préférence destinés à un milieu hydrocarboné ou fluoré, alors que les mélanges de diméthylsiloxane oxyéthylène/oxypropyléné sont de préférence destinés à un milieu siliconé.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi le mica recouvert de titane ou d'oxychlorure de bismuth, le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth. On peut également utiliser les pigments interférentiels, notamment à cristaux liquides ou multicouches.

Les colorants hydrosolubles sont par exemple le jus de betterave, le bleu de méthylène.

La composition selon l'invention peut comprendre au moins une charge, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon@) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la Société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La première et/ou la deuxième composition de l'invention peut, en outre, contenir un ou plusieurs actifs cosmétiques ou dermatologiques tels que ceux classiquement utilisés.

Comme actifs cosmétiques, dermatologiques, hygiéniques ou pharmaceutiques, utilisables dans la composition de l'invention, on peut citer les hydratants, vitamines, acides gras essentiels, sphingolipides, filtres solaires. Ces actifs sont utilisés en quantité habituelle pour l'homme de l'art et notamment à des concentrations de 0 à 20 % et notamment de 0,001 à 15 % du poids total de la première ou deuxième composition.

La composition peut comprendre, en outre, tout autre additif usuellement utilisé dans de telles compositions, tel que de l'eau, des antioxydants, des parfums, des conservateurs et des huiles essentielles.

Bien entendu l'homme du métier veillera à choisir ce ou ces éventuels composés complémentaires, et/ou leur quantité, de manière telle que les propriétés avantageuses de la composition ne soient pas, ou substantiellement pas, altérées par l'adjonction envisagée.

Les compositions du produit peuvent se présenter sous forme d'un produit coulé et par exemple sous la forme d'un stick ou bâton, ou sous la forme de coupelle utilisable par contact direct ou à l'éponge. En particulier, elles trouvent une application en tant que fond de teint coulé, fard à joues ou à paupières coulé, rouge à lèvres, base ou baume de soin pour les lèvres, produit anti-cernes, vernis à ongles. Elles peuvent aussi se présenter sous forme d'une pâte souple ou encore de gel, de crème plus ou moins fluide, ou de liquide conditionné dans un tube.

Les compositions du produit selon l'invention peuvent constituer notamment une composition cosmétique de soin pour le visage, pour le cou, pour les mains ou pour le corps (par exemple crème de soin, huile solaire, gel corporel), une composition de maquillage (par exemple gel de maquillage, crème, stick) ou une composition de bronzage artificiel ou de protection de la peau.

Les compositions du produit selon l'invention peuvent se présenter sous la forme d'une composition de soin de la peau et/ou des phanères ou sous forme d'une composition de protection solaire, d'hygiène corporelle, notamment sous forme de déodorant. Elles se présentent alors notamment sous forme non colorée. Elles peuvent alors être utilisées comme base de soin pour la peau, les phanères ou les lèvres (baumes à lèvres, protégeant les lèvres du froid et/ou du soleil et/ou du vent, crème de soin pour la peau, les ongles ou les cheveux).

Par cosmétiquement acceptable, on entend au sens de l'invention une composition d'aspect, d'odeur et de toucher agréables.

Chaque composition du produit selon l'invention peut se présenter sous toute forme galénique normalement utilisée pour une application topique et notamment sous forme d'une solution huileuse ou aqueuse, d'un gel huileux ou aqueux, d'une émulsion huile-dans-eau ou eau-dans-huile, d'une émulsion multiple, d'une dispersion d'huile dans de l'eau grâce à des vésicules, les vésicules étant situés à l'interface huile/eau, ou d'une poudre. Chaque composition peut être fluide ou solide.

Selon un mode de mise oeuvre, la première ou la seconde composition, ou les deux, sont à phase grasse continue et de préférence sous forme anhydre et peuvent contenir moins de 5% d'eau, et encore mieux moins de 1% d'eau par rapport au poids total de la première ou seconde composition.

Chaque première et seconde composition peut avoir l'aspect d'une lotion, d'une crème, d'une pommade, d'une pâte souple, d'un onguent, d'un solide coulé ou moulé et notamment en stick ou en coupelle, ou encore de solide compacté.

Chaque composition peut être conditionnée séparément dans un même article de conditionnement par exemple dans un stylo bi-compartimenté, la composition de base étant délivrée par une extrémité du stylo et la composition du dessus étant délivrée par l'autre extrémité du stylo, chaque extrémité étant fermée notamment de façon étanche par un capuchon.

Alternativement, chacune des compositions peut être conditionnée dans un article de conditionnement différent.

De préférence, la composition qui est appliquée en première couche est sous forme liquide ou pâteuse, ce qui est hautement souhaitable dans le cas d'un rouge à lèvres ou d'un eyeliner.

Le produit selon l'invention peut être avantageusement utilisé pour le maquillage de la peau et/ou des lèvres et/ou des phanères selon la nature des ingrédients utilisés. En particulier, le produit de l'invention peut se présenter sous forme de fond de teint solide, de bâton ou pâte de rouge à lèvres, de produit anti-cernes, ou contours des yeux, d'eye liner, de mascara, d'ombre à paupières, de produit de maquillage du corps ou encore un produit de coloration de la peau.

Avantageusement, la deuxième composition présente des propriétés de soin, de brillance ou de transparence.

L'invention a encore pour objet un produit à lèvres, un vernis, un mascara, un fond de teint, un tatouage, un fard à joues ou à paupières comprenant une première et une seconde compositions telles que décrites précédemment.

Les compositions du produit de l'invention peuvent être obtenues par chauffage des différents constituants à la température de fusion des cires les plus élevées, puis coulage du mélange fondu dans un moule (coupelle ou doigt de gant). Elles peuvent aussi être obtenues par extrusion comme décrit dans la demande EP-A-0 667 146.

L'invention est illustrée plus en détail dans les exemples suivants. Les pourcentages sont des pourcentages en poids.

**Exemple 2 :**

Première composition : Stick de rouge à lèvres

Dans un poêlon, sous agitation Rayneri, on introduit les cires, les pâtes pigmentaires et l'ester de saccharose, on se place à 105°C, on laisse tourner 30 minutes, puis on ajoute les nacres, on ajoute ensuite la dispersion de polymère et le parfum, on laisse tourner 10 minutes, puis on coule dans un moule à 42°C. On place le moule au congélateur et on démoule lorsque le moule est à environ 4°C.

| **Ingrédients** | **% massique** |
|---|---|
| Pigments | 8,20 |
| Hydrogenated polyisobutene (Parleam) | 5,18 |
| Polyhydroxystearic acid (Octacare DSPOL300, Avecia) | 0,21 |
| C30-C50 alcohols (Performacol 550 Alcohol, New Phase Tecnologies) | 2 |
| Polyethylene (Polywax 500, Bareco) | 10 |
| Sucrose acetate isobutyrate (Eastman SAIB Special, Eastman Chemical) | 5 |
| Polymère de l'exemple 1 | 68,82 |
| parfum | qs |

Deuxième composition : Stick incolore pour les lèvres

| | % en poids |
|---|---|
| Huile de silicone (PDMS) DC200 de Dow Corning (5 cSt) | 25 |
| DIMETHICONE (and) DIMETHICONOL D2-9085 de Dow Corning (1550 cSt) | 61 |
| TRIFLUOROPROPYL DIMETHICONE (100 cSt) X22-819 de Shin Etsu | 1 |
| C30-45 ALKYL DIMETHICONE (SF 1642 de GE Bayer Silicone) | 5 |
| Cire de POLYETHYLENE (PM en poids 500) | 8 |

L'huile de silicone, la diméthiconol et la diméthicone fluorée sont mélangées à chaud jusqu'à former un mélange homogène. On ajoute ensuite la $C_{30}$-$C_{45}$ alkyl diméthicone dans le mélange précédent porté à 110°C. La cire de polyéthylène est ensuite ajoutée peu à peu jusqu'à obtention d'un mélange homogène. Le mélange est refroidi à 90-95°C puis versé dans les moules, qui sont placés à -20°C pendant trente minutes. On procède enfin au démoulage des sticks.

La tenue au repas du produit de maquillage des lèvres constitué des deux compositions décrites précédemment est mesurée selon la méthode décrite précédemment. La valeur est de 98,95 (incertitude de mesure 0,48).
La brillance du produit de maquillage des lèvres mesurée selon la méthode décrite précédemment est égale à 129,8.
Le transfert du produit de maquillage des lèvres mesuré selon la méthode décrite précédemment est égale à 5,7.

## Revendications

1. Produit cosmétique comprenant une première et une seconde compositions, la première composition contenant u ne dispersion, dans une phase grasse liquide, de particules d'un polymère éthylénique greffé, et la seconde composition distincte de la première comprenant un milieu cosmétiquement acceptable.

2. Produit selon la revendication précédente, **caractérisé par le fait que** le polymère éthylénique greffé comprend un squelette éthylénique insoluble dans ladite phase grasse liquide et des chaînes latérales liées de manière covalente audit squelette et solubles dans ladite phase grasse liquide.

3. Produit selon la revendication 1 ou 2, **caractérisé par le fait que** le polymère éthylénique greffé est un polymère acrylique greffé.

4. Produit selon l'une quelconque des revendications précédentes, **caractérisé par le fait que** le polymère éthylénique est dispersé en l'absence de stabilisant additionnel en surface des particules de polymère greffé.

5. Produit selon l'une quelconque des revendications précédentes, **caractérisé par le fait que** le polymère éthylénique greffé en dispersion est un polymère acrylique susceptible d'être obtenu par polymérisation radicalaire dans un milieu organique de polymérisation :

- d'au moins un monomère acrylique, et éventuellement d'au moins un monomère additionnel vinylique non acrylique, pour former ledit squelette insoluble ; et
- d'au moins un macromonomère comportant un groupe terminal polymérisable pour former les chaînes latérales, ledit macromonomère ayant une masse moléculaire moyenne en poids supérieure ou égale à 200 et la teneur en macromonomère polymérisé représentant de 0,05 à 20% en poids du polymère.

**6.** Produit selon la revendication précédente, **caractérisé par le fait que** le monomère acrylique est choisi parmi, seul ou en mélange, les monomères suivants, ainsi que leurs sels :

- (i) les (méth)acrylates de formule :

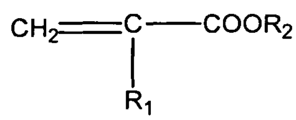

dans laquelle :

- $R_1$ désigne un atome d'hydrogène ou un groupe méthyle ;
- $R_2$ représente un groupe choisi parmi :
    - un groupe alkyle linéaire ou ramifié, comprenant de 1 à 6 atomes de carbone, ledit groupe pouvant comporter dans sa chaîne un ou plusieurs hétéroatomes choisis parmi O, N et S; et/ou pouvant comporter un ou plusieurs substituants choisis parmi -OH, les a tomes d'halogène (F, Cl, Br, I) et -NR'R" avec R' e t R" identiques ou différents choisis parmi les alkyles linéaires ou ramifiés en $C_1$-$C_4$; et/ou pouvant être substitué par au moins un groupe polyoxyalkylène, notamment polyoxyéthylène et/ou polyoxypropylène, ledit groupe polyoxyalkylène étant constitué par la répétition de 5 à 30 motifs oxyalkylène ;
    - un groupe alkyle cyclique comprenant de 3 à 6 atomes de carbone, ledit groupe pouvant comporter dans sa chaîne un ou plusieurs hétéroatomes choisis parmi O, N et S, et/ou pouvant comporter un ou plusieurs substituants choisis parmi OH et les atomes d'halogène (F, Cl, Br, I) ;

- (ii) les (méth)acrylamides de formule :

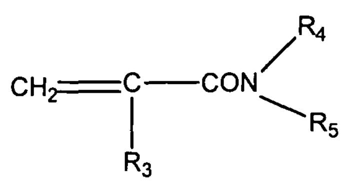

dans laquelle :

- $R_3$ désigne un atome d'hydrogène ou un groupe méthyle ;
- $R_4$ et $R_5$, identiques ou différents, représentent un atome d'hydrogène ou un groupe alkyle, linéaire ou ramifié, comportant de 1 à 6 atomes de carbone, pouvant comporter un ou plusieurs substituants choisis parmi -OH, les atomes d'halogène (F, Cl, Br, I) et -NR'R" avec R' et R" identiques ou différents choisis parmi les alkyles linéaires ou ramifiés en $C_1$-$C_4$;ou
- $R_4$ représente un atome d'hydrogène et $R_5$ représente un groupe 1,1-diméthyl-3-oxobutyle ;

- (iii) les monomères (méth)acryliques comprenant au moins une fonction acide carboxylique, phosphorique ou sulfonique, tels que l'acide acrylique, l'acide méthacrylique, l'acide acrylamidopropanesulfonique.

**7.** Produit selon la revendication 5 ou 6, **caractérisé par le fait que** le monomère acrylique est choisi parmi les (méth)acrylates de méthyle, d'éthyle, de propyle, de butyle, d'isobutyle; les (méth)acrylates de méthoxyéthyle ou d'éthoxyéthyle ; le méthacrylate de trifluoroéthyle; le méthacrylate de diméthylaminoéthyle, le méthacrylate de diéthylaminoéthyle, le (méth)acrylate de 2-hydroxypropyle, le (méth)acrylate de 2-hydroxyéthyle ; le diméthylaminopropylméthacrylamide; l'acide (méth)acrylique et leurs sels.

8. Produit selon l'une quelconque des revendications 5 à 7 , **caractérisé par le fait que** le monomère acrylique est choisi parmi l'acrylate de méthyle, l'acrylate de méthoxyéthyle, le méthacrylate de méthyle, le méthacrylate de 2-hydroxyéthyle, l'acide (méth)acrylique, le méthacrylate de diméthylaminoéthyle, et leurs mélanges.

9. Produit selon l'une quelconque des revendications précédentes, **caractérisé par le fait que** le polymère greffé comprend de l'acide (méth)acrylique.

10. Produit selon l'une quelconque des revendications 5 à 9, **caractérisé par le fait que** les monomères acryliques comprennent au moins l'acide (méth)acrylique et au moins un monomère choisi parmi les (méth)acrylates et les (méth)acrylamides décrits aux points (i) et (ii) dans la revendication 6.

11. Produit selon l'une quelconque des revendications 5 à 10, **caractérisé par le fait que** les monomères acryliques comprennent au moins l'acide (méth)acrylique et au moins un monomère choisi parmi les (méth)acrylates d'alkyle en $C_1$-$C_3$.

12. Produit selon l'une quelconque des revendications 9 à 11, **caractérisé par le fait que** l'acide (méth)acrylique est présent en une teneur d'au moins 5 % en poids, par rapport au poids total du polymère, notamment allant de 5 % à 80 % en poids, de préférence d'au moins 10 % en poids, notamment allant de 10 % en poids à 70 % en poids, préférentiellement d'au moins 15 % en poids, notamment allant de 15 % à 60 % en poids.

13. Produit selon l'une quelconque des revendications précédentes, **caractérisé par le fait que** le polymère acrylique greffé ne contient pas de monomère vinylique non acrylique additionnel.

14. Produit selon l'une quelconque des revendications 5 à 12, **caractérisé par le fait que** le polymère acrylique greffé est susceptible d'être obtenu par polymérisation radicalaire d'un ou plusieurs monomère(s) acrylique(s) et d'un ou plusieurs monomère(s) additionnel(s) vinylique(s) non acrylique(s), et dudit macromonomère.

15. Produit selon l'une quelconque des revendications 5 à 12 et 14, **caractérisé par le fait que** les monomères additionnels vinyliques non acryliques sont choisis parmi :

- les esters vinylique de formule : $R_6$-COO-CH=$CH_2$
  dans laquelle $R_6$ représente un groupe alkyle linéaire ou ramifié, comprenant de 1 à 6 atomes, ou un groupe alkyle cyclique comportant de 3 à 6 atomes de carbone et/ou un groupe aromatique, par exemple de type benzénique, anthracénique, et naphtalénique ;
- les monomères vinyliques non acryliques comprenant au moins une fonction acide carboxylique, phosphorique ou sulfonique, tels que l'acide crotonique, l'anhydride maléique, l'acide itaconique, l'acide fumarique, l'acide maléique, l'acide styrènesulfonique, l'acide vinylbenzoïque, l'acide vinylphosphorique et les sels de ceux-ci ;
- les monomères vinyliques non acryliques comprenant au moins une fonction amine tertiaire, tels que la 2-vinylpyridine, la 4-vinylpyridine ;
- et leurs mélanges.

16. Produit selon l'une quelconque des revendications 5 à 15, **caractérisé par le fait que** les monomères acryliques représentent de 50 à 100 % en poids, de préférence de 60 à 100 % en poids , préférentiellement de 70 à 100 % en poids du mélange monomères acryliques + monomères vinyliques non acryliques éventuels.

17. Produit selon l'une quelconque des revendications 5 à 16, **caractérisé par le fait que** le macromonomère comporte à une des extrémités de la chaîne un groupe terminal polymérisable choisi parmi un groupe vinyle ou un groupe (méth)acrylate, et de préférence un groupe (méth)acrylate.

18. Produit selon l'une quelconque des revendications 5 à 17, **caractérisé par le fait que** le macromonomère a une masse moléculaire moyenne en poids supérieure ou égale à 300, préférentiellement supérieure ou égale à 500, et plus préférentiellement supérieure à 600.

19. Produit selon l'une quelconque des revendications 5 à 18, **caractérisé par le fait que** le macromonomère a une masse moléculaire moyenne en poids (Mw) allant de 200 à 100 000, de préférence allant de 500 à 50 000, préférentiellement allant de 800 à 20 000, plus préférentiellement allant de 800 à 10000, et encore plus préférentiellement allant de 800 à 6000.

**20.** Produit selon l'une quelconque des revendications 5 à 19, **caractérisé par le fait que** le macromonomère polymérisé représente de 0,1 à 15 % en poids du poids total du polymère, de préférence de 0,2 à 10 % en poids, et préférentiellement de 0,3 à 8 % en poids,

**21.** Produit selon l'une quelconque des revendications précédentes, **caractérisé par le fait que** la phase grasse liquide comprend un composé organique liquide choisi parmi :

- les composés organiques liquides ayant un paramètre de solubilité global selon l'espace de solubilité de Hansen inférieur ou égal à 18 $(MPa)^{1/2}$,
- les monoalcools ayant un paramètre de solubilité global selon l'espace de solubilité de Hansen inférieur ou égal à 20 $(MPa)^{1/2}$; et
- leurs mélanges.

**22.** Produit selon l'une quelconque des revendications précédentes, **caractérisé par le fait que** la phase grasse liquide comprend une huile volatile.

**23.** Produit selon l'une quelconque des revendications précédentes, **caractérisé par le fait que** la phase grasse liquide est une phase grasse liquide non siliconée.

**24.** Produit selon la revendication précédente, **caractérisé par le fait que** la phase grasse liquide non siliconée est constituée d'au moins 50% en poids d'au moins un composé liquide organique non siliconé choisi parmi :

- les composés liquides organiques non s iliconés ayant un paramètre de solubilité global selon l'espace de solubilité de Hansen inférieur ou égal à 18 $(MPa)^{1/2}$;
- les monoalcools liquides ayant un paramètre de solubilité global selon l'espace de solubilité de Hansen inférieur ou égal à 20 $(MPa)^{1/2}$; et
- leurs mélanges.

**25.** Produit selon la revendication 23 ou 24, **caractérisé par le fait que** la phase grasse liquide non siliconée comprend moins de 50 % en poids de composés organiques liquides siliconés ayant un paramètre de solubilité global selon l'espace de solubilité de Hansen inférieur ou égal à 18 $(MPa)^{1/2}$.

**26.** Produit selon la revendication 23 ou 24, **caractérisé par le fait que** la phase grasse liquide non siliconée ne contient pas de composés organiques liquides siliconés.

**27.** Produit selon l'une quelconque des revendications 23 à 26, **caractérisé par le fait que** le macromonomère est un macromonomère carboné.

**28.** Produit selon la revendication précédente, **caractérisé par le fait que** le macromonomère carboné est choisi parmi :

- (i) les homopolymères et les copolymères d'acrylate ou de méthacrylate d'alkyle en $C_8$-$C_{22}$ linéaire ou ramifié et ayant un groupe terminal polymérisable choisi parmi les groupes vinyle ou (méth)acrylate ;
- (ii) les polyoléfines ayant un groupe terminal à insaturation éthylénique polymérisable.

**29.** Produit selon la revendication 27 ou 28, **caractérisé par le fait que** le macromonomère carboné est choisi parmi :

- (i) les macromonomères de poly(acrylate d'éthyl-2 hexyle) à extrémité mono(méth)acrylate ; les macromonomères de poly(acrylate de dodécyle) à extrémité mono(méth)acrylate ; les macromonomères de poly(méthacrylate de dodécyle) ; les macromonomères de poly(acrylate de stéaryle) à extrémité mono(méth)acrylate ; les macromonomères de poly (méthacrylate de stéaryle) à extrémité mono(méth)acrylate ;

- (ii) les macromonomères de polyéthylène, les macromonomères de polypropylène, les macromonomères de copolymère polyéthylène/polypropylène, les macromonomères de copolymère polyéthylène/polybutylène, les macromonomères de polyisobutylène, les macromonomères de polybutadiène, les macromonomères de polyisoprène, les macromonomères de polybutadiène, les macromonomères de poly(éthylène/butylène)-polyisoprène, ces macromonomères ayant un groupement terminal (méth)acrylate.

**30.** Produit selon l'une quelconque des revendications 27 à 29, **caractérisé par le fait que** le macromonomère carboné est choisi parmi :

- (i) les macromonomères de poly(acrylate d'éthyl-2-hexyle) à extrémité mono(méth)acrylate, les macromonomères de poly(acrylate de dodécyle) à extrémité mono(méth)acrylate ;
- (ii) le méthacrylate de poly(éthylène/butylène).

**31.** Produit selon l'une quelconque des revendications 27 à 30, **caractérisé par le fait que** le polymère greffé est choisi parmi les polymères obtenus par polymérisation :

- de l'acrylate de méthyle et du macromonomère polyéthylène/polybutylène à groupement terminal méthacrylate, en particulier dans un solvant choisi parmi l'isododécane, l'isononylisononanoate, l'octyldodécanol , le malate de diisostéaryle, un benzoate d'alkyl $C_{12}$-$C_{15}$ ;
- de l'acrylate de méthoxyéthyle et du macromonomère polyéthylène/polybutylène à groupement terminal méthacrylate, en particulier dans l'isododécane ;
- des monomères a crylate de méthyle / méthacrylate de méthyle et du macromonomère polyéthylène/polybutylène à groupement terminal méthacrylate, en particulier dans l'isododécane ;
- des monomères acrylate de méthyle / acide acrylique et du macromonomère polyéthylène/polybutylène à groupement terminal méthacrylate, en particulier dans l'isododécane ;
- des monomères acrylate de méthyle / méthacrylate de diméthylaminoéthyle et du macromonomère polyéthylène/polybutylène à groupement terminal méthacrylate, en particulier dans l'isododécane ;
- des monomères acrylate de méthyle / méthacrylate de 2-hydroxyéthyle et du macromonomère polyéthylène/polybutylène à groupement terminal méthacrylate, en particulier dans l'isododécane.

**32.** Produit selon l'une quelconque des revendications 23 à 31, **caractérisé par le fait que** le polymère greffé est un polymère greffé non siliconé.

**33.** Produit selon la revendication précédente, **caractérisé par le fait que** le polymère greffé non siliconé contient majoritairement un macromonomère carboné et contenant éventuellement au plus 7 % en poids de macromonomère siliconé, du poids total du polymère.

**34.** Produit selon la revendication 32 ou 33, **caractérisé par le fait que** le polymère greffé siliconé est exempt de macromonomère carboné.

**35.** Produit selon l'une quelconque des revendications 1 à 22, **caractérisé par le fait que** la phase grasse liquide est une phase grasse liquide siliconée.

**36.** Produit selon la revendication précédente, **caractérisé par le fait que** la phase grasse liquide siliconée est constituée d'au moins 50 % en poids d'au moins un composé liquide organique siliconé choisi parmi les composés liquides organiques siliconés ayant un paramètre de solubilité global selon l'espace de solubilité de Hansen inférieur ou égal à 18 $(MPa)^{1/2}$.

**37.** Produit selon la revendication 25 ou 36, **caractérisé par** le fait le composé liquide organique siliconé comprend une huile siliconée volatile.

**38.** Produit selon la revendication précédente, **caractérisé par le fait que** l'huile siliconée volatile est choisie parmi l'octaméthylcyclotétrasiloxane, le déca-méthylcyclopentasiloxane, le dodécaméthylcyclohexasiloxane, l'heptaméthyl-hexyltrisiloxane, l'heptaméthyloctyltrisiloxane, l'octaméthyltrisiloxane, le décaméthyltétrasiloxane, et leurs mélanges.

**39.** Produit selon la revendication 25 ou 36, **caractérisé par** le fait le composé liquide organique siliconé comprend une huile siliconée non volatile.

**40.** Produit selon la revendication précédente, **caractérisé par le fait que** l'huile siliconée non volatile est choisie parmi les polydialkylsiloxanes non volatils; les polydiméthylsiloxanes comportant des groupements alkyle, alcoxy ou phényle, pendant ou en bout de chaîne siliconée, groupements ayant de 2 à 24 atomes de carbone; les silicones

phénylées ; les polysiloxanes modifiés par des acides gras (notamment en $C_8$-$C_{20}$), des alcools gras (notamment en $C_8$-$C_{20}$) ou des polyoxyalkylènes (notamment polyoxyéthyléne et/ou polyoxypropylène); les polysiloxanes aminées ; les polysiloxanes à groupement hydroxyles; les polysiloxanes fluorés comportant un groupement fluoré pendant ou en bout de chaîne siliconée ayant de 1 à 12 atomes de carbone dont tout ou partie des hydrogène sont substitués par des atomes de fluor ; et leurs mélanges.

**41.** Produit selon l'une quelconque des revendications 35 à 40, **caractérisé par le fait que** la phase grasse liquide comprend moins de 50 % en poids de composés organiques liquides non siliconés.

**42.** Produit selon la revendication 24 ou 41, **caractérisé par le fait que** le composé organique liquide non siliconé est choisi parmi les composés organiques liquides non siliconés ayant un paramètre de solubilité global selon l'espace de solubilité de Hansen inférieur à 18 $(MPa)^{1/2}$; les monoalcools liquides ayant un paramètre de solubilité global selon l'espace de solubilité de Hansen inférieur ou égal à 20 $(MPa)^{1/2}$; et leurs mélanges.

**43.** Produit selon la revendication 24 ou 41, **caractérisé par le fait que** le composé liquide organique non siliconé ayant un paramètre de solubilité global selon l'espace de solubilité de Hansen inférieur ou égal à 18 $(MPa)^{1/2}$ est choisi parmi les huiles carbonées, hydrocarbonées, fluorées, seules ou en mélange ; les alcanes linéaires, ramifiés et/ou cycliques, éventuellement volatils; les esters et notamment les esters linéaires, ramifiés ou cycliques, ayant au moins 6 atomes de carbone; les cétones et notamment les cétones ayant au moins 6 atomes de carbone; les éthers et notamment les éthers ayant au moins 6 atomes de carbone.

**44.** Produit selon la revendication 24 ou 41, **caractérisé par le fait que** les monoalcools ayant un paramètre de solubilité global selon l'espace de solubilité de Hansen inférieur ou égal à 20 $(MPa)^{1/2}$ sont choisis parmi les monoalcools gras aliphatiques ayant 6 à 30 atomes de carbone, la chaîne hydrocarbonée ne comportant pas de groupement de substitution, et notamment l'alcool oléique, le décanol, et l'alcool linoléique.

**45.** Produit selon la revendication 22, **caractérisé par le fait que** l'huile volatile est une huile volatile non siliconée.

**46.** Produit selon la revendication précédente, **caractérisé par le fait que** l'huile volatile non siliconée est choisie parmi l'isododécane, l'isodécane, l'isohexadécane.

**47.** Produit selon l'une quelconque des revendications 35 à 40, **caractérisé par le fait que** la phase grasse liquide ne contient pas de composés organiques liquides non siliconés.

**48.** Produit selon l'une quelconque des revendications 35 à 47, **caractérisé par le fait que** le macromonomère est un macromonomère siliconé.

**49.** Produit selon la revendication précédente, **caractérisé par le fait que** le macromonomère siliconé est un macromonomère organopolysiloxane, et de préférence un macromonomère polydiméthylsiloxane.

**50.** Produit selon l'une quelconque des revendications 48 à 49, **caractérisé par le fait que** le macromonomère est choisi parmi les polydiméthylsiloxanes à groupement terminal mono (méth)acrylate, et notamment les monométhacryloxypropyl polydiméthylsiloxanes.

**51.** Produit selon l'une quelconque des revendications 48 à 50, **caractérisé par le fait que** le macromonomère siliconé est choisi parmi les macromonomères de formule (II) suivante :

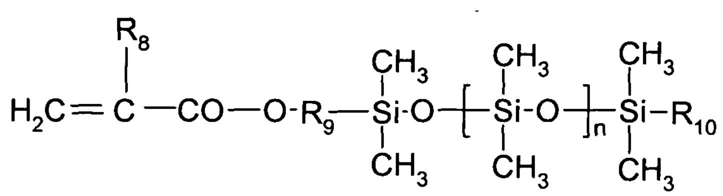

(II)

dans laquelle $R_8$ désigne un atome d'hydrogène ou un groupement méthyle ; $R_9$ désigne un groupe hydrocarboné divalent ayant de 1 à 10 atomes de carbone et contient éventuellement une ou deux liaisons éther -O- ; R10 désigne un groupe alkyl ayant de 1 à 10 atomes de carbone, notamment de 2 à 8 atomes de carbone ; n désigne

un nombre entier allant de 1 à 300, de préférence allant de 3 à 200, et préférentiellement allant de 5 à 100.

**52.** Produit selon l'une quelconque des revendications 35 à 51, **caractérisé par le fait que** le polymère acrylique greffé est susceptible d'être obtenu par polymérisation radicalaire dans le milieu de polymérisation :

- d'un monomère acrylique principal choisi parmi les (méth)acrylates d'alkyle en $C_1$-$C_3$, seul ou en mélange, et éventuellement d'un ou plusieurs monomères acryliques additionnels choisis parmi l'acide acrylique, l'acide méthacrylique et les (méth)acrylates d'alkyle de formule (I) :

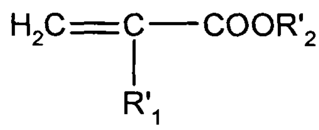

(I)

dans laquelle :

- R'$_1$ désigne un atome d'hydrogène ou un groupe méthyle ;
- R'$_2$ représente :

  . un groupe alkyle linéaire ou ramifié comprenant de 1 à 6 atomes de carbone, ledit groupe comportant dans sa chaîne un ou plusieurs atomes d'oxygène et/ou comportant un ou plusieurs substituants choisis parmi -OH, les atomes d'halogène (F, Cl, Br, I) et -NR'R" avec R' et R", identiques ou différents, choisis parmi les alkyles linéaires ou ramifiés en $C_1$-$C_3$ ;
  . un groupe alkyle cyclique comprenant de 3 à 6 atomes de carbone, ledit groupe pouvant comporter dans sa chaîne un ou plusieurs atomes d'oxygène et/ou pouvant comporter un ou plusieurs substituants choisis parmi OH et les atomes d'halogène (F, Cl, Br, I) ;

et leurs sels, pour former ledit squelette insoluble ; et

- et d'un macromonomère siliconé.

**53.** Produit selon la revendication précédente, **caractérisé par le fait que** R'$_2$ désigne un groupe choisi parmi les groupes méthoxyéthyle, éthoxyéthyle, trifluoroéthyle; 2-hydroxyéthyle, 2-hydroxypropyle, diméthylamino-éthyle, diéthyl-aminoéthyle, diméthylaminopropyle.

**54.** Produit selon la revendication 52 ou 53, **caractérisé par le fait que** le monomère acrylique principal est choisi parmi le (méth)acrylate de méthyle, le (méth)acrylate d'éthyle, le (méth)acrylate de n-propyle, le (méth)acrylate d'iso-propyle, et leurs mélanges.

**55.** Produit selon l'une quelconque des revendications 52 à 54, **caractérisé par le fait que** le monomère acrylique principal est choisi parmi l'acrylate de méthyle, le méthacrylate de méthyle, le méthacrylate d'éthyle.

**56.** Produit selon la revendication 52, **caractérisé par le fait que** le monomère acrylique additionnel est choisi parmi l'acide (méth)acrylique, le (méth)acryla6e de méthoxyéthyle, le (méth)acrylate d'éthoxyéthyle, le méthacrylate de trifluoroéthyle, le méthacrylate de diméthylaminoéthyle, le méthacrylate de diéthylaminoéthyle, le (méth)acrylate de 2-hydroxypropyle, le (méth)acrylate de 2-hydroxyéthyle, et leurs sels.

**57.** Produit selon la revendication 52 ou 56, **caractérisé par le fait que** le monomère acrylique additionnel est choisi parmi l'acide acrylique, l'acide méthacrylique.

**58.** Produit selon l'une quelconque des revendications 35 à 57, **caractérisé par le fait que** le polymère greffé est choisi parmi les polymères obtenus par polymérisation :

- de l'acrylate de méthyle et du macromonomère monométhacryloxypropylpolydiméthylsiloxane ayant un poids moléculaire moyen en poids allant de 800 à 6000, en particulier dans le décaméthylcyclopentasiloxane ou le phényltriméthicone ;
- de l'acrylate de méthyle, d'acide acrylique et du macromonomère monométhacryloxypropylpolydiméthylsiloxane ayant un poids moléculaire moyen en poids allant de 800 à 6000, en particulier dans le décaméthylcyclopentasiloxane ou le phényltriméthicone.

**59.** Produit selon l'une quelconque des revendications 35 à 58, **caractérisé par le fait que** le polymère greffé est un polymère greffé siliconé.

**60.** Produit selon la revendication précédente, **caractérisé par le fait que** le polymère greffé siliconé contient majoritairement un macromonomère siliconé et contenant éventuellement au plus 7 % en poids de macromonomère carboné, du poids total du polymère.

**61.** Produit selon la revendication 59 ou 60, **caractérisé par le fait que** le polymère greffé siliconé est exempt de macromonomère carboné.

**62.** Produit selon l'une quelconque des revendications précédentes, **caractérisé par le fait que** le polymère greffé a une masse moléculaire moyenne en poids (Mw) comprise entre 10 000 et 300 000, notamment entre 20 000 et 200 000, mieux encore entre 25 000 et 150 000.

**63.** Produit selon l'une quelconque des revendications précédentes, **caractérisé par le fait que** les particules de polymère greffé ont une taille moyenne allant de 10 à 400 nm, de préférence allant de 20 à 200 nm.

**64.** Produit selon l'une quelconque des revendications précédentes, **caractérisé par le fait que** le polymère éthylénique greffé est un polymère filmogène.

**65.** Produit selon l'une quelconque des revendications précédentes, **caractérisé par le fait que** le polymère greffé est présent en une teneur allant de 0,1 % à 70 % en poids, par rapport au poids total de la composition, de préférence allant de 0,5 % à 50 % en poids, et préférentiellement allant de 1 % à 40 % en poids.

**66.** Produit cosmétique selon l'une quelconque des revendications précédentes, **caractérisé par le fait que** l'une au moins des première et seconde compositions se présente sous forme de pâte ou de stick.

**67.** Produit selon l'une des revendications précédentes, **caractérisé en ce que** la seconde composition est transparente.

**68.** Produit selon l'une des revendications précédentes, **caractérisé en ce que** la seconde composition contient moins de 5% de pigments, de préférence moins de 2%, de préférence encore moins de 1 %.

**69.** Produit selon l'une des revendications précédentes, **caractérisé en ce que** son indice de transfert est inférieur à 40, de préférence inférieur ou égal à 30, de préférence inférieur ou égal à 20, de préférence inférieur ou égal à 15, de préférence inférieur ou égal à 10, de préférence, inférieur ou égal à 5, et de préférence encore inférieur ou égal à 2 sur 100.

**70.** Produit selon l'une des revendications précédentes, **caractérisé en ce que** sa brillance moyenne mesurée à 20° est supérieure ou égale à 30, de préférence supérieure ou égale à 40, de préférence supérieure ou égale à 50, de préférence supérieure ou égale à 60.

**71.** Produit selon l'une des revendications précédentes, **caractérisé en ce que** son indice de tenue est supérieur ou égal à 80 %, de préférence supérieur ou égal à 85 %, de préférence inférieur ou égal à 90 %, de préférence supérieur ou égal à 95 %.

**72.** Produit selon l'une des revendications précédentes, **caractérisé en ce que** la deuxième composition est choisie de telle manière à améliorer au moins une propriété cosmétique de la première composition, lorsque celle-ci est appliquée seule sur ladite matière kératinique.

**73.** Produit selon l'une des revendications précédentes, **caractérisé en ce que** le milieu cosmétiquement acceptable de la seconde composition comprend un polymère siliconé choisi parmi les diméthiconols, les polydiméthylsiloxanes et leurs mélanges.

**74.** Produit selon l'une des revendications précédentes, **caractérisé en ce que** la première composition et/ou la

deuxième composition contient un agent de coloration choisi parmi les colorants liposolubles, les colorants hydrosolubles, les pigments, les nacres et leurs mélanges.

**75.** Produit selon l'une des revendications précédentes, **caractérisé en ce que** la première et/ou la seconde composition est sous forme d'un liquide anhydre, d'un stick anhydre ou d'une émulsion.

**76.** Produit selon l'une des revendications précédentes, **caractérisé en ce qu'**il se présente sous la forme d'un fond de teint, d'un fard à joues ou à paupières, d'un rouge à lèvres, d'un produit ayant des propriétés de soin, d'un mascara, d'un eye-liner, d'un vernis à ongles, d'un produit anti-cernes, ou d'un produit de maquillage du corps.

**77.** Procédé de maquillage de la peau et/ou des lèvres et/ou des phanères consistant à appliquer sur la peau, les lèvres et/ou les phanères une première couche d'une première composition comprenant une dispersion, dans une phase grasse liquide, de particules d'un polymère éthylénique greffé et un agent de coloration, puis à appliquer, sur tout ou partie de ladite première couche, une seconde couche d'une seconde composition comprenant un milieu cosmétiquement acceptable.

**78.** Procédé de maquillage selon la revendication précédente **caractérisé en ce qu'**il comprend en outre une étape de séchage au moins partiel de la première composition avant application de la seconde.

**79.** Kit de maquillage comprenant un produit selon l'une des revendications 1 à 76, chacune des première et seconde compositions étant conditionnées de manière séparée dans des premier et second compartiments.

**80.** Kit de maquillage selon la revendication 79 **caractérisé en ce que** les premier et second compartiments sont formés à l'intérieur d'un même récipient.

**81.** Kit de maquillage selon la revendication 79 **caractérisé en ce que** les premier et second compartiments sont formés dans deux récipients distincts.

**82.** Utilisation d'un produit cosmétique comprenant première et une seconde compositions, la première composition comprenant une dispersion, dans une phase grasse liquide, de particules d'un polymère éthylénique greffé, et la seconde composition comprenant un milieu cosmétiquement acceptable, l'une au moins desdites compositions contenant un agent de coloration, pour conférer à la peau et/ou les lèvres et/ou les phanères un maquillage confortable, brillant, non transfert et/ou non migrant et/ou de bonne tenue de la couleur et/ou de bonne tenue de la brillance.

**Office européen des brevets**

# RAPPORT DE RECHERCHE EUROPEENNE

Numéro de la demande
EP 05 29 0962

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| D,X | FR 2 823 101 A (L'OREAL) 11 octobre 2002 (2002-10-11) * le document en entier * ----- | 1-82 | A61K7/021 |
| X | FR 2 823 102 A (L'OREAL) 11 octobre 2002 (2002-10-11) * le document en entier * ----- | 1-82 | |
| X | US 2003/039621 A1 (ARNAUD PASCAL ET AL) 27 février 2003 (2003-02-27) * le document en entier * ----- | 1-82 | |
| D,A | PATENT ABSTRACTS OF JAPAN vol. 017, no. 674 (C-1140), 10 décembre 1993 (1993-12-10) & JP 05 221829 A (KOSE CORP), 31 août 1993 (1993-08-31) * abrégé * ----- | 1-82 | |
| D,A | WO 02/067877 A (REVLON CONSUMER PRODUCTS CORPORATION) 6 septembre 2002 (2002-09-06) * revendications 1-20 * ----- | 1-82 | DOMAINES TECHNIQUES RECHERCHES (Int.Cl.7) |
| | | | A61K |

Le présent rapport a été établi pour toutes les revendications

1

| Lieu de la recherche | Date d'achèvement de la recherche | Examinateur |
|---|---|---|
| Munich | 6 septembre 2005 | Yon, J-M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..........
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 05 29 0962

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

06-09-2005

| Document brevet cité au rapport de recherche | Date de publication | Membre(s) de la famille de brevet(s) | Date de publication |
|---|---|---|---|
| FR 2823101 A | 11-10-2002 | FR 2823101 A1 | 11-10-2002 |
| | | BR 0201262 A | 11-03-2003 |
| | | CA 2380789 A1 | 10-10-2002 |
| | | CN 1382431 A | 04-12-2002 |
| | | EP 1249223 A1 | 16-10-2002 |
| | | FR 2823102 A1 | 11-10-2002 |
| | | JP 2002322020 A | 08-11-2002 |
| | | US 2005129641 A1 | 16-06-2005 |
| | | US 2003039621 A1 | 27-02-2003 |
| FR 2823102 A | 11-10-2002 | FR 2823101 A1 | 11-10-2002 |
| | | FR 2823102 A1 | 11-10-2002 |
| | | BR 0201262 A | 11-03-2003 |
| | | CA 2380789 A1 | 10-10-2002 |
| | | CN 1382431 A | 04-12-2002 |
| | | EP 1249223 A1 | 16-10-2002 |
| | | JP 2002322020 A | 08-11-2002 |
| | | US 2005129641 A1 | 16-06-2005 |
| | | US 2003039621 A1 | 27-02-2003 |
| US 2003039621 A1 | 27-02-2003 | FR 2823101 A1 | 11-10-2002 |
| | | FR 2823102 A1 | 11-10-2002 |
| | | US 2005129641 A1 | 16-06-2005 |
| | | BR 0201262 A | 11-03-2003 |
| | | CA 2380789 A1 | 10-10-2002 |
| | | CN 1382431 A | 04-12-2002 |
| | | EP 1249223 A1 | 16-10-2002 |
| | | JP 2002322020 A | 08-11-2002 |
| JP 05221829 A | 31-08-1993 | JP 3098604 B2 | 16-10-2000 |
| WO 02067877 A | 06-09-2002 | CA 2439371 A1 | 06-09-2002 |
| | | EP 1389085 A2 | 18-02-2004 |
| | | MX PA03007637 A | 21-04-2004 |
| | | WO 02067877 A2 | 06-09-2002 |
| | | US 2002159960 A1 | 31-10-2002 |

EPO FORM P0460

Pour tout renseignement concernant cette annexe : voir Journal Officiel de l'Office européen des brevets, No.12/82